(19) Europäisches Patentamt
European Patent Office
Office européen des brevets

(11) Publication number: 0 373 461 A2

# (12) EUROPEAN PATENT APPLICATION

(21) Application number: 89122281.2

(22) Date of filing: 02.12.89

(51) Int. Cl.5: C07D 413/04, C07D 417/04, A01N 43/74, C07D 471/02, C07D 487/02, C07D 413/14, C07D 417/14, //(C07D471/02, 233:00,211:00),(C07D487/02, 249:00,237:00)

(30) Priority: 14.12.88 JP 313956/88

(43) Date of publication of application: 20.06.90 Bulletin 90/25

(84) Designated Contracting States: BE CH DE FR GB IT LI NL

(71) Applicant: NIHON TOKUSHU NOYAKU SEIZO K.K.
7-1, Nihonbashi Honcho 2-Chome Chuo-ku
Tokyo(JP)

(72) Inventor: Kume, Toyohiko
6-7-8, Asahigaoka
Hino-shi Tokyo(JP)
Inventor: Goto, Toshio
3454-21, Honmachida
Machida-shi Tokyo(JP)
Inventor: Tamura, Tatsuo
1-7-30, Hanenaka Hamura-machi
Nishitama-gun Tokyo(JP)
Inventor: Kamochi, Atsumi
2-24-10, Higashi-Toyoda
Hino-shi Tokyo(JP)
Inventor: Shibuya, Katsuhiko
39-15, Namiki-cho
Hachioji-shi Tokyo(JP)
Inventor: Miyauchi, Hiroshi
39-15, Namiki-cho
Hachioji-shi Tokyo(JP)

(74) Representative: Schumacher, Günter, Dr. et al
c/o Bayer AG Konzernverwaltung RP
Patentabteilung
D-5090 Leverkusen 1 Bayerwerk(DE)

## (54) Benzazoles.

(57) Novel benzazoles of the formula (I)

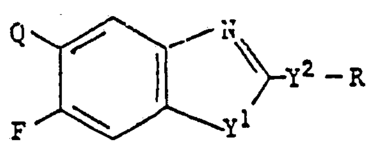

wherein Q represents

EP 0 373 461 A2

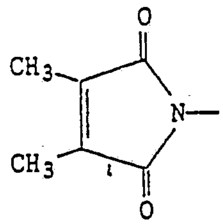 , 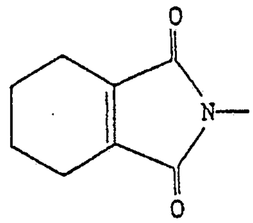 or 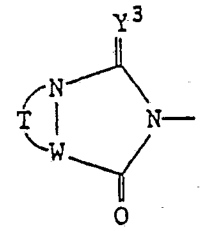

and the use of the new compounds as herbicides.

## Benzazoles

The present invention relates to novel benzazoles, to several processes for their preparation, and to their use as herbicides.

It has already been disclosed that certain benzazoles are useful as herbicides (see Japanese Laid-Open Patent Publication No. 14711/1988).

There have now been found novel benzazoles of the formula (I)

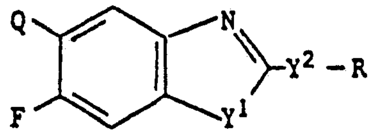

(I)

wherein Q represents

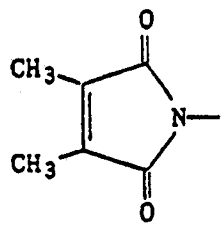 , 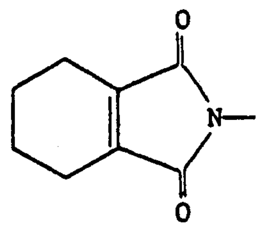 or 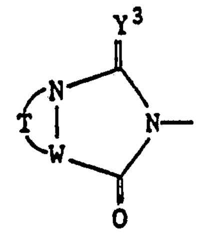

in which T represents $-(CH_2)_4-$ or $-CH_2-CH=CH-CH_2-$,
W represents CH or N, and
$Y^3$ represents O or S,
$Y^1$ and $Y^2$ each represent O or S, and
R represents an optionally by halogen substituted $C_{1-10}$ alkyl group, a cycloalkylmethyl group having $C_{3-6}$ cycloalkyl, which is optionally substituted by a $C_{1-3}$ alkyl group, or represents $C_{3-10}$ alkoxyalkyl,-$CH_2COOR^1$ or $-CH_2CONR^2R^3$, wherein $R^1$ represents a $C_{1-6}$ alkyl group or a $C_{5-6}$ cycloalkyl group, $R^2$ and $R^3$ each represent a $C_{1-6}$ alkyl group or $R^2$ and $R^3$ together may form a five-or six-membered ring that may comprise oxygen or $N-CH_3$, or
R represents a methyl group substituted by a five- or six-membered heterocyclic group, wherein said heterocyclic group may be substitutable with halogen, $C_{1-3}$ alkyl and/or $C_{1-3}$ alkoxy,

Benzazoles of the formula (I) are obtained when

a) in the case Q represents

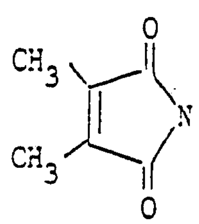 or 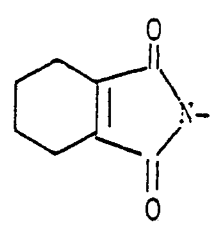 , then

Q is replaced by 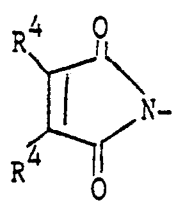 , in which

$R^4$ is methyl or tetramethylene;
compounds of the formula (II)

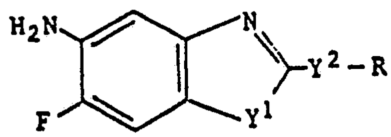

(II)

wherein $Y^1$, $Y^2$ and R are abovementioned, are reacted with compound of the formula (III)

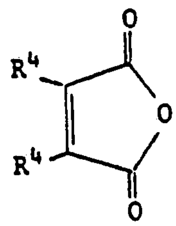

(III)

wherein $R^4$ is abovementioned, in the presence of inert solvents, or

b) in the case where Q is

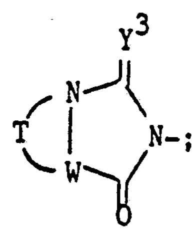

compounds of the formula (IV)

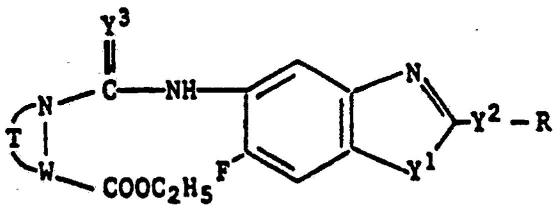

(IV)

wherein $Y^1$, $Y^2$, R, T, W and $Y^3$ are abovementioned, are condensed in the presence of inert solvents.

The novel benzazoles exhibit powerful herbicidal properties.

Surprisingly, the benzazoles according to the invention exhibit a substantially greater herbicidal action than those known from the prior art, for instance, aforementioned Japanese Laid-Open Patent Publication No. 14711/1985.

Among the benzazoles according to the invention, of the formula (I), preferred compounds are those wherein

Q represents

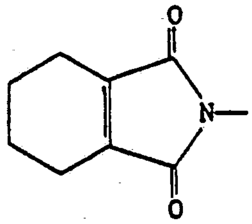

or

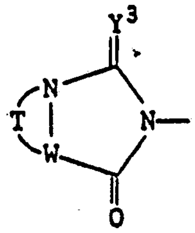

in which T represents $-(CH_2)_4-$, and

W represents CH or N,
$Y^3$ represents O or S,
$Y^1$ represents S,
$Y^2$ represents O,
and R presents an optionally by halogen substituted $C_{1-6}$ alkyl group, a cycloalkylmethyl group having optionally by methyl substituted $C_{3-6}$ cycloalkyl, a $C_{1-3}$ alkoxy-$C_{2-3}$ alkyl group, $-CH_2COOR^1$ or $-CH_2CONR^2R^3$,
wherein $R^1$ represents a $C_{1-4}$ alkyl group or cyclopentyl group,
R2 and $R^3$ each represent a $C_{1-4}$ alkyl group or $R^2$ and $R^3$ together may form a five- or six-membered ring that may comprise oxygen or $N\text{-}CH_3$, or
R represents a methyl group substituted by a five-membered heterocyclic group that may be substituted with chlorine, methyl and/or methoxy, wherein said heterocyclic group may comprise one to two nitrogen atoms and further one of oxygen atom, sulfur atom or nitrogen atom, or a six-membered heterocyclic group that may comprise one to two nitrogen atoms and optionally chlorine substituent.

Very particularly preferred benzazoles of the formula (I) are those wherein Q represents

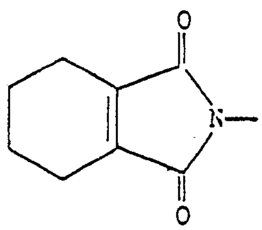

or

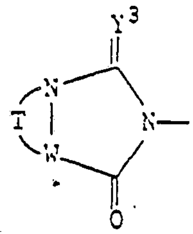

in which T represents $-(CH_2)_4-$, W represents CH or N and
$Y^3$ represents S,
$Y^1$ represents S, $Y^2$ represents O and
R represents methyl, ethyl, in each case straight chain or branched propyl, butyl, pentyl, hexyl or heptyl, which are optionally substituted by fluorine, methoxymethyl, methoxyethyl, ethoxymethyl, ethoxyethyl, methoxycarbonylmethyl, ethoxycarbonylmethyl, dimethylaminocarbonylmethyl, diethylaminocarbonylmethyl or dipropylaminocarbonylmethyl.

As the concrete examples of the five- and six-membered heterocyclic rings defined under the aforementioned symbol R may be mentioned, for example, thiazole, isoxazole, 1,2,4-triazole, 1,2,4-oxadiazole, 1,2,5-oxadiazole, 1,2,5-thiadiazole, pyridine, pyrazine and so on.
In particular R represents methyl, ethyl, in each case straight chain or branched propyl, butyl, pentyl, hexyl or heptyl which are optionally substituted by fluorine.

Specifically, the compounds of the following formulae may be mentioned:

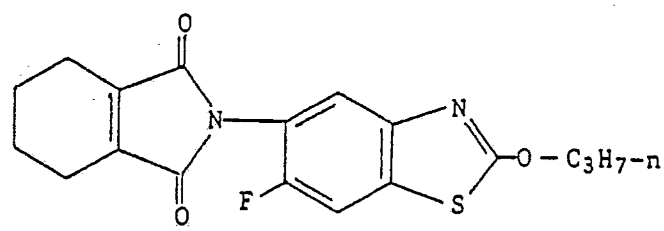

,

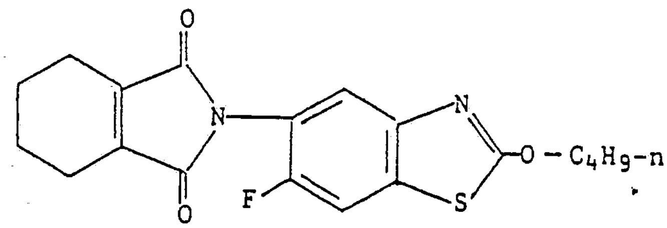

,

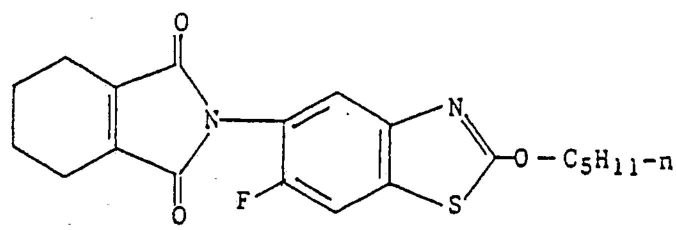

, and

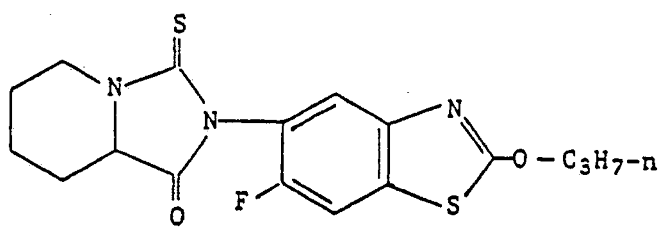

,

If, for example, 5-amino-2-butoxy-6-fluorobenzothiazole and 3,4,5,6-tetrahydrophthalic anhydride are used as starting materials, the course of the reaction can be represented by the following equatiion:

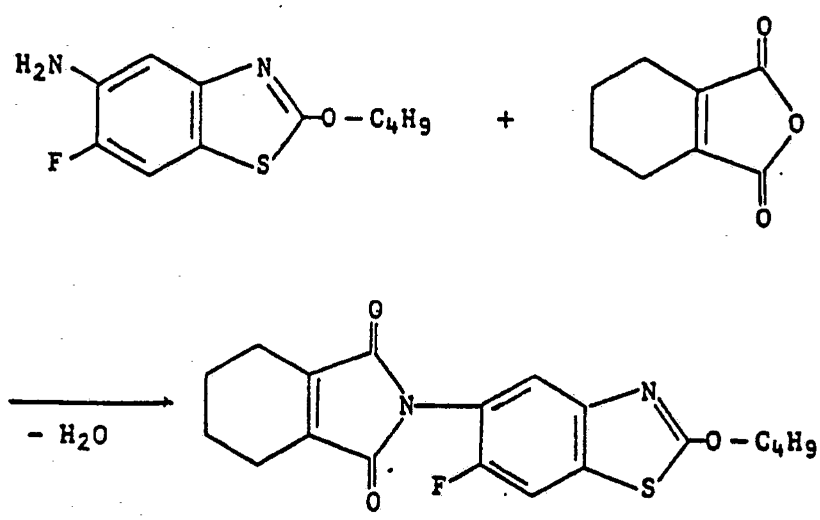


If, for example, 5-(2-ethoxycarbonyl-hexahydropyridazin-1-yl carbonylamino)-6-fluoro-2-propoxyben-

zothiazole and sodium methoxide are used as starting materials, the course of the reaction can be represented by the following equation:

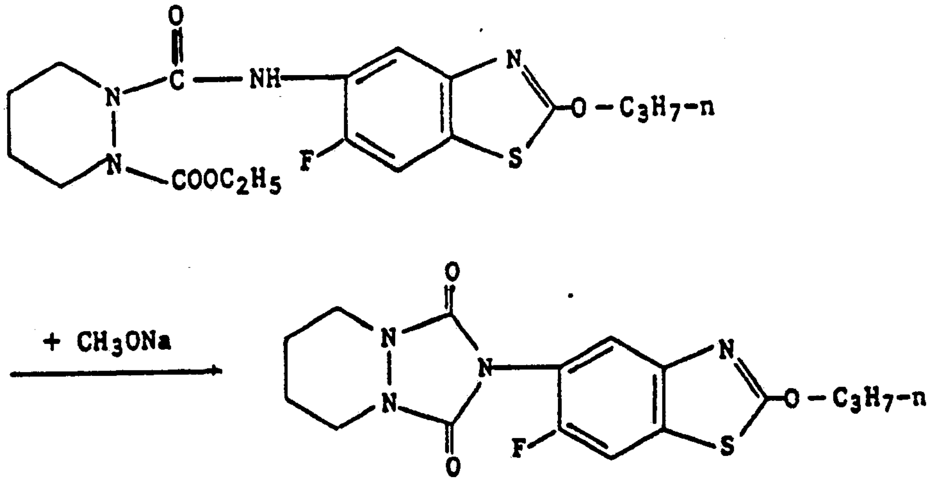


Referring to the compounds represented by the formula (II) employed as starting materials in the process a), the symbols $Y^1$, $Y^2$ and R in this formula have the same meanings as stated before. Furthermore, in the formula (II), the symbols $Y^1$, $Y^2$ and R preferably have the same preferred meanings as stated before.

The compounds represented by the formula (II) include the known compounds disclosed by Japanese Patent Application No. 153466-1988 as well as novel compounds which have not yet been disclosed by any publication.

Further, the compound (II) mentioned above can be prepared according to the process that was disclosed by the above-mentioned Japanese Patent Application No. 153466-1988.

As the concrete examples of the compounds represented by the general formula (II), may be mentioned:
5-amino-6-fluoro-2-propoxybenzothiazole,
5-amino-2-butoxy-6-fluorobenzothiazole, and
5-amino-6-fluoro-2-pentyloxybenzothiazole, etc.

The compounds represented by the general formula (III) employed in the above-mentioned process a) are known in themselves and concretely include 2,3-dimethylmaleic anhydride and 3,4,5,6-tetrahydrophthalic anhydride.

Regarding the starting material represented by the general formula (IV) employed as material in the process b), the substituents are those as defined under the afore-mentioned symbols $Y^1$, $Y^2$, R, T, W and $Y^3$.

In the general formula (IV), the symbols $Y^1$, $Y^2$, R, T, W and $Y^3$ have the same preferable meanings as mentioned before.

The compounds represented by the general formula (IV) are novel compounds and can be prepared by process c), for example, wherein a compound represented by the general formula

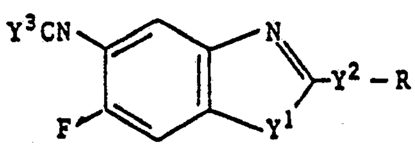 (V)

wherein $Y^1$, $Y^2$, R and $Y^3$ have the same meanings as mentioned before,
is reacted with a compound represented by the general formula

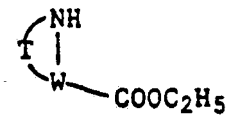

(VI)

wherein T and W have the same meanings as mentioned before.

The compounds represented by the above-mentioned general formula (V) are novel compounds which can be prepared by process d), for example, wherein a compound represented by the afore-mentioned general formula (II) is reacted with one of the compounds selected from the group consisting of phosgene, trichloromethyl chloroformate and thiophosgene.

In carrying out the above-mentioned process d), an isocyanate derivative of the compound (V) can be synthesized by the process disclosed in Organic Synthesis, Collective, vol. 2, page 453 or J. Am. Chem. Soc. vol. 72, page 1888 and an isothiocyanate derivative of the compound (V) can be synthesized by the process disclosed in J. Org. Chem., 1953, page 1052 or J. Chem. Soc., 1927, page 1186.

Regarding the above-mentioned process c), the reaction partner represented by the general formula (VI) includes, as the concrete examples, ethyl pipecolinate, 1-ethoxycarbonylhexahydropyridazine, and 1-ethoxycarbonyl-1,2,3,6-tetrahydropyridazine, all of which are the compounds disclosed by Japanese Patent Application No. 258462-1987.

The above-mentioned process c) can be carried out in accordance with the process disclosed by Agr. Biol. Chem., vol. 40, No. 4, page 745.

As appropriate diluents for carrying out the process a), any kind of inert organic solvents can be mentioned.

As the examples of such solvents, use may be made preferably of organic acids such as, for example, acetic acid, propionic acid, etc.

When carrying out the above-mentioned process a), the respective starting materials are refluxed under heating in acetic acid, for example, according to the above-mentioned process disclosed in Agr. Biol. Chem., vol. 40, page 745.

In carrying out the above-mentioned process b), use may be made, as appropriate diluents, of alcohols such as, for example, ethanol, isopropanol, tert-butanol, methyl cellosolve, etc., dioxane, N,N-dimethylformamide, dimethylsulfoxide, sulfolane, etc.

The process b) can be carried out at a temperature from about 50 °C to about 130 °C, for example, preferably from about 70 °C to about 90 °C.

The above-mentioned process b) can be carried out according to the process disclosed in the above-mentioned Agr. Chem. Biol., vol. 40, No. 45, page 745, wherein, in general, about 0.01 to 0.5 equivalents of sodium ethoxide are reacted with one equivalent of the compound represented by the general formula (IV) in the presence of one of the above-mentioned solvents and at a reaction temperature in the range mentioned above for a period from 0.5 to 16 hours.

In carrying out the process b), use is made, as base, of triethyl amine, sodium hydroxide, potassium hydroxide, or sodium methoxide, etc., in place of sodium ethoxide, in the above.

Further, in the preparation of compounds of the formula (I) according to process (b), the compound represented by the general formula (IV) that has been prepared in the preceding reaction stage designated as the process c) may be used as it is in the process b). Namely, the process c) and process b) may be continuously carried out.

The active compounds according to the invention can be used as defoliants, desiccants, agents for destroying broad-leaved palnts and, especially, as weedkillers. By weeds, in the broadest sense, there are to be understood all plants which grow in locations where they are undesired. Whether the substances according to the invention act as total or selective herbicides depends essentially on the amount used.

The active compouns according to the invention can be used, for example, in connection with the following plants:

<u>Dicotyledon weeds of the genera:</u>

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsia, Papaver and Centaurea.

Dicotyledon cultures of the genera:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis and Cucurbita.

Monocotyledon weeds of the genera:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus and Apera.

Monocotyledon cultures of the genera:

Oryza, Zea, Triticum, Hordeum, Avena, Secale, Sorghum, Panicum, Saccharum, Ananas, Asparagus and Allium.

However, the use of the active compounds according to the invention is in no way restricted to these genera, but also extends in the same manner to other plants.

The compounds are suitable, depending on the concentration, for the total combating of weeds, for example on industrial terrain and rail tracks, and on paths and squares with or without tree plantings. Equally, the compounds can be emplyed for combating weeds in perennial cultures, for example afforestations, decorative tree plantings, orchards, vineyards, citrus groves, nut orchards, banana plantations, coffee plantations, tea plantations, rubber plantations, oil palm plantations, cocoa plantations, soft fruit plantings and hopfields, and for the slective combating of weeds in annual cultures.

The active compounds can be converted into the customary formulations, such as solutions, emulsions, wettable powders, suspensions, powders, foams, pastes, granules, aerosols, natural and synthetic materials impregnated with active compound, very fine capsules in polymeric substances, coating compositions for use on seed, and formulations used with burning equipment, such as fumigating cartridges, fumigating cans and fumigating coils, as well as ULV cold mist and warm mist formulations.

These formulations may be produced in known manner, for example by mixing the active compounds with extenders, that is to say liquid or liquefied gaseous or solid diluents or carriers, optionally with the use of surface-active agents, that is to say emulsifying agents and/or dispersing agents and/or foam-forming agents. In the case of the use of water as an extender, organic solvents can, for example, also be used as auxiliary solvents.

As liquid solvents diluents or carriers, there are suitable in the main, aromatic hydrocarbons, such as xylene, toluene or alkyl napthalenes, chlorinated aromatic or chlorinated aliphatic hydrocarbons, such as chlorobenzenes, chloroethylenes or methylene chloride, aliphatic hydrocarbons, such as cyclohexane or paraffins, for example mineral oil fractions, alcohols, such as butanol or glycol as well as their ethers and esters, ketones, such as acetone, methyl ethyl ketone, methyl isobutyl ketone or cyclohexanone, or strongly polar solvents, such as dimethylformamide and dimethyl-sulphoxide, as well as water.

By liquefied gaseous diluents or carriers are meant liquids which would be gaseous at normal temperature and under normal pressure, for example aerosol propellants, such as halogenated hydrocarbons as well as butane, propane, nitrogen and carbon dioxide.

As solid carriers there may be used ground natural minerals, such as kaolins, clays, talc, chalk, quartz, attapulgite, montmorillonite or diatomaceous earth, and ground synthetic minerals, such as highly-dispersed silicic acid, alumina and silicates. As solid carriers for granules there may be used crushed and fractionated natural rocks such as calcite, marble, pumice, sepiolite and dolomite, as well as synthetic granules of inorganic and organic meals, and granules or organic material such as sawdust, coconut shells, maize cobs and tobacco stalks.

As emulsifying and/or foam-forming agents there may be used non-ionic and anionic emulsifiers, such as polyoxyethylene-fatty acid esters, polyoxyethylene-fatty alcohol ethers, for example alkylaryl polyglycol ethers, alkyl sulphonates, alkyl sulphates, aryl sulphonates as well as albumin hydrolysis products. Dispersing agents include, for example, lignin sulphite waste liquors and methylcellulose.

Adhesive such as carboxymethylcellulose and natural and synthetic polymers in the form of powders, granules or latices, such as gum arabic, polyvinyl alcohol and polyvinyl acetate, can be used in the formulation.

It is possible to use colorants such as inorganic pigments, for example iron oxide, titanium oxide and

Prussian Blue, and organic dyestuffs, such as alizarin dyestuffs, azo dyestoffs or metal phthalocyanine dyestuffs, and trace nutrients, such as salts of iron, manganese boron, copper, cobalt, molybdenum and zinc.

The formulation in general contain from 0.1 to 95 per cent by weight of active compound, preferably from 0.5 to 90 per cent by weight.

The active compounds according to the invention, as such or in the form of their formulations, can also be used, for combating weeds, as mixtures with known herbicides, finished formulations or tank mixes being possible.

Mixtures with other known active compounds, such as herbicides, fungicides, insecticides, acaricides, nematicides, bird repellants, plant nutrients and agents which improve soil structure, are also possible.

The active compounds can be used as such, in the form of their formulations or in the use forms prepared therefrom by further dilution, such as ready-to-use solutions, suspensions, emulsions, powders, pastes and granules. They are used in the customary manner, for example by watering, spraying, atomising or scattering.

The active compounds according to the invention can be applied either before or after emergence of the plants.

They can also be incorporated into the soil before sowing. They are used, in particular, after emergence of the plants.

The amount of active compound used can vary within a substantial range. It depends essentially on the nature of the desired effect. In general, the amounts used are between 0.0001 and 3 kg of active compound per hectare of soil surface, preferably between 0.001 and 2 kg per ha.

The preparation and use of the active compounds according to the invention can be seen from the following examples.

Preparative Examples

Example 1

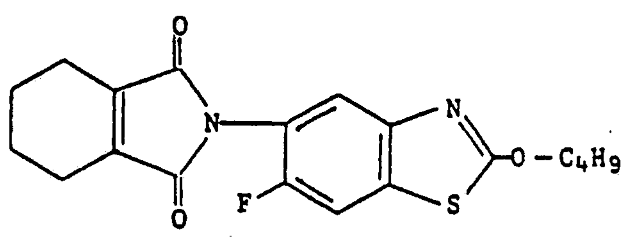

To a mixture of 5-amino-2-butoxy-6-fluorobenzothiazole (2.5 g) and acetic acid (30 ml) was added 3,4,5,6-tetrahydrophthalic anhydride (1.6 g) and the mixture was stirred at 20 to 30 °C for a period of thirty minutes, followed by heating under reflux for two hours. The residue obtained by distilling the acetic acid off from the mixture under reduced pressure was purified through silicagel column chromatography (eluent = chloroform) to obtain the aimed 2-butoxy-6-fluoro-5-(4,5,6,7-tetrahydro-2H-isoindole-1,3-dion-2-yl)-benzothiazole (3.0 g).
$n_D^{20}$ 1.5840

Example 2

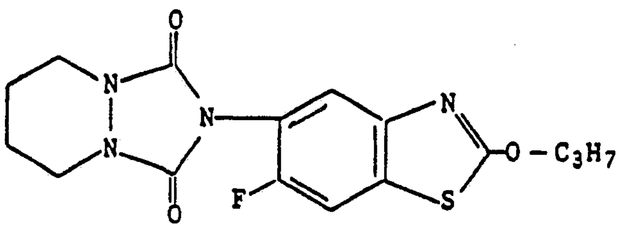

A mixture was composed of 6-fluoro-5-isocyanato-2-propoxybenzothiazole (2.0 g), 1-ethoxycarbonylhexahydropyridazine (1.2 g), dioxane (20 ml), toluene (20 ml) and catalytic amount of triethylamine was stirred at 10 to 20 °C for five hours. The solvent was distilled off from the reaction mixture under reduced pressure to obtain white crystals (2.7 g). The crystals were subjected to each of NMR and IR spectro analyses, thereby enabling to identify them to be a compound having a melting point in the range from 146 to 150 °C and represented by the following chemical formula (compound No. IV-1):

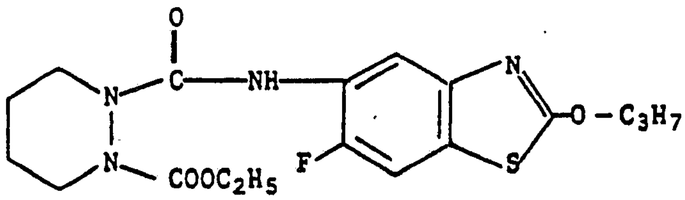

To this compound obtained in the yield of 2.6 g was added methanol (30 ml) that comprised catalytic amount of sodium methoxide,and the resulting mixture was heated under reflux for three hours, followed by distilling off the solvent under reduced pressure. The resulting residue was purified through silicagel column chromatography (eluent = chloroform) to obtain the aimed 6-fluoro-2-propoxy-5-(1,2-tetramethylene-1,2,4-triazolidine-3,5-dion-4-yl)benzothiazole (1.8 g) in porous crystals.

Example 3

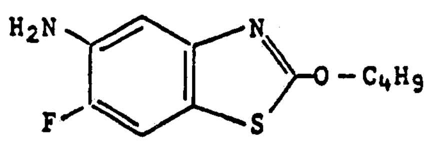

A solution of oily 6-fluoro-2-butoxy-5-nitrobenzothiazole (8.5 g) dissolved in tetrahydrofuran (20 ml) was dropwise added to a mixture consisting of iron powder (8.8 g), water (8 ml) and acetic acid (4.0 g) at 70 to 80 °C and, after completion of the dropwise addition, the mixture was stirred at 80 to 85 °C for one hour.

Then tetrahydrofuran was removed from the reaction mixture under distillation and after the addition of ethyl acetate (200 ml) thereto, it was stirred and filtered. The thus obtained ethyl acetate layer was washed with water and dried with anhydrous sodium sulfate. A solid residue obtained by removing the ethyl acetate from the thus dried reaction mixture under distillation at reduced pressure was dissolved in a minimum amount of n-hexane under refluxing, followed by filtration of crystals forming upon a slow cooling thereof at 15 to 25 °C so as to obtain the aimed 5-amino-6-fluoro-2-butoxybenzothiazole (4.5 g) having a melting point in the range of from 101 to 103 °C.

Referential Example 1

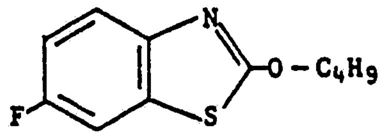

Powder of 85% potassium hydroxide (6.3 g) was dissolved in n-butanol (80 ml) and to the solution was added portionwise 2-chloro-6-fluorobenzothiazole (12.0 g) at 0.5 to 5° C. After stirring at 20 to 35° C for one and a half hours, the n-butanol was distilled off under reduced pressure and to the thus obtained residue were added toluene (40 ml) and water (20 ml), followed by stirring thereof and separation of an organic layer therefrom. The separated organic layer was washed with water, dried with anhydrous sodium sulfate evaporated under reduced pressure to obtain 6-fluoro-2-butoxybenzothiazole (12.2 g).
This compound, without any further purification, was used in the subsequent reaction.

Referential Example 2

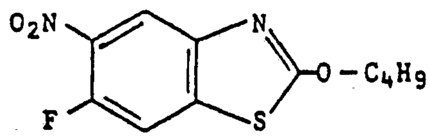

6-fluoro-2-butoxybenzothiazole (8.9 g) was added to 95% sulfuric acid (100 g) at 0 to 10° C, followed by stirring for 30 minutes. Then, 98% nitric acid (3.1 g) was dropwise added to the mixture at 0 to 5° C followed by one hour stirring while the temperature being kept at 0 to 5° C.

The reaction mixture was poured onto water (300 g) and the resulting oily substance was extracted with toluene and the extract was washed with water, saturated sodium bicarbonate and water, in that order, followed by drying with anhydrous sodium sulfate. After distilling off the toluene under reduced pressure, oily substance (8.8 g) was obtained.

By GLC analysis, the content in the oily substance of the principal component, viz., 6-fluoro-2-butoxy-5-nitrobenzothiazole was determined to be in the range from 72 to 77%.

Referential Example 3 (compound No. V-1)

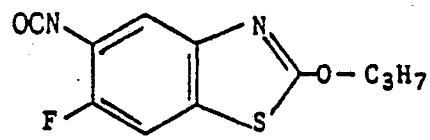

To a mixture of 5-amino-6-fluoro-2-propoxybenzothiazole (3.0 g) and dioxane (50 ml) was added dropwise trichloromethyl chloroformate (2.2 g) at 10 to 20° C, and the mixture was heated under reflux until the generation of gaseous hydrogen chloride ceased. The reaction product was concentrated and dried under reduced pressure to obtain the aimed 6-fluoro-5-isocyanato-2-propoxybenzothiazole (3.0 g).

This compound, without further purification, was used in the subsequent reaction.

Referential Example 4 (compound No. V-2)

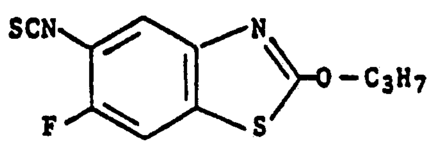

To a mixture consisting of thiophosgene (3.4 g) and water (60 ml) was added dropwise a solution of 5-amino-6-fluoro-2-propoxy-benzothiazole (6.0 g) in chloroform (60 ml) at 10 to 20 °C, followed by two hour stirring while the temperature being kept at 10 to 20 °C.

To the reaction mixture was added portionwise sodium bicarbonate at 5 to 10 °C so as to render it to become weak alkaline. After the weak alkalinity of the mixture was confirmed upon a further 20 minutes stirring, a chloroform layer was separated therefrom, while aqueous layer was further extracted twice therefrom with chloroform (30 ml x 2).

The thus obtained chloroform layers were combined, washed with water, dried with anhydrous sodium sulfate followed by removal of chloroform therefrom, to obtain the aimed 6-fluoro-5-isothiocyanato-2-propoxy-benzothiazole (6.2 g) having a melting point in the range from 65.5 to 66.5 °C.

Referential Example 5

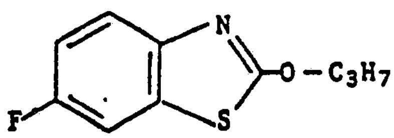

To n-propanol (40 ml) was added potassium tert. butoxide (3.4 g) at 0 to 5 °C, followed by one hour stirring.

Then, to the reaction liquid was added 2-methylthio-6-fluorobenzothiazole (5.0 g) portionwise at 0 to 5 °C, followed by two hours stirring at 20 to 35 °C. Then reaction liquid was treated in the manner similar to Referential Example 1 to obtain the aimed 6-fluoro-2-propoxybenzothiazole (3.8 g).

This compound, without further purification, was used in the subsequent reaction.

The compounds according to the present invention which can be obtained by the same processes as those of the afore-mentioned Examples 1 and 2 are demonstrated in the following tables.

In the tables, those compounds which were obtained in the foregoing Examples 1 and 2 are also included.

Table 1 relates to the compounds represented by the general formula (I) defined as:

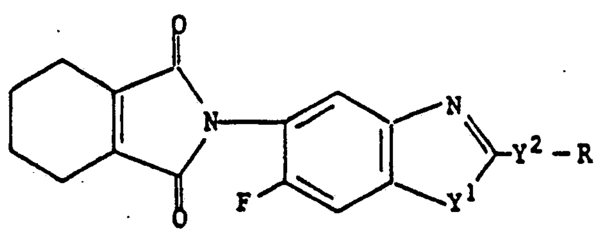

Table 2 relates to the compounds represented by the general formula (I) defined as:

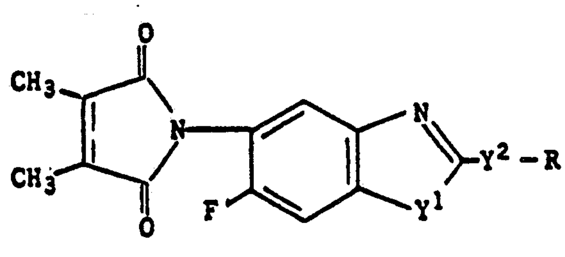

Table 3 relates to the compounds represented by the general formula (I) defined as:

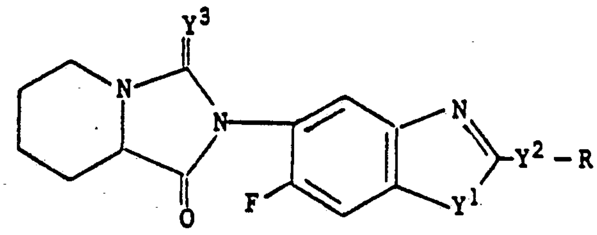

Table 4 relates to the compounds represented by the general formula (I) defined as:

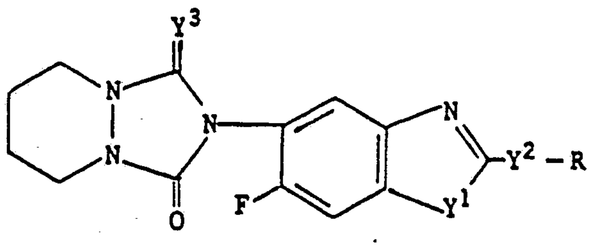

, and

Table 5 relates to the compounds represented by the general formula (I) defined as:

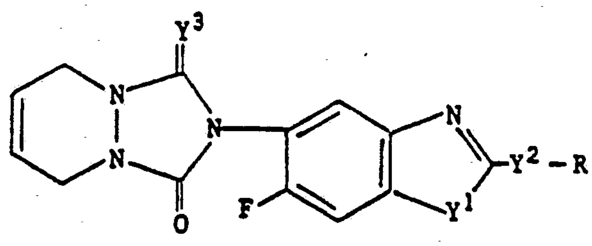

Table 1

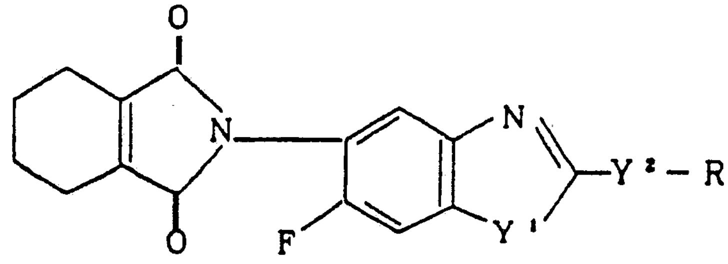

| Compound No. | $Y^1$ | $Y^2$ | R | |
|---|---|---|---|---|
| 1 | O | O | $CH_3$ | |
| 2 | S | O | $CH_3$ | mp. 118 - 121 ℃ |
| 3 | S | S | $CH_3$ | |
| 4 | O | O | $C_2H_5$ | |
| 5 | O | S | $C_2H_5$ | |
| 6 | S | O | $C_2H_5$ | mp. 124 - 127 ℃ |
| 7 | S | S | $C_2H_5$ | |
| 8 | O | O | $C_3H_7$-n | |
| 9 | O | S | $C_3H_7$-n | mp. 112 - 116 ℃ |
| 10 | S | O | $C_3H_7$-n | mp.112.5 - 113.5℃ |
| 11 | S | S | $C_3H_7$-n | mp. 95 - 100 ℃ |
| 12 | O | O | $C_4H_9$-n | |
| 13 | O | S | $C_4H_9$-n | |
| 14 | S | O | $C_4H_9$-n | $n_D^{22}$ 1.5775 |
| 15 | S | S | $C_4H_9$-n | |
| 16 | O | O | $C_4H_9$-iso | |

Table 1 - Continuation

| Compound No. | $Y^1$ | $Y^2$ | R | |
|---|---|---|---|---|
| 17 | S | O | $C_4H_9$-iso | $n_D^{20}$ 1.5750 |
| 18 | S | S | $C_4H_9$-iso | |
| 19 | O | O | $C_5H_{11}$-n | |
| 20 | O | S | $C_5H_{11}$-n | |
| 21 | S | O | $C_5H_{11}$-n | $n_D^{22}$ 1.5800 |
| 22 | S | S | $C_5H_{11}$-n | |
| 23 | O | O | $CH_2CH_2CH(CH_3)_2$ | |
| 24 | S | O | $CH_2CH_2CH(CH_3)_2$ | $n_D^{20}$ 1.5750 |
| 25 | S | S | $CH_2CH_2CH(CH_3)_2$ | |
| 26 | O | O | $CH_2CH(CH_3)-CH_2CH_3$ | |
| 27 | S | O | $CH_2CH(CH_3)-CH_2CH_3$ | $n_D^{20}$ 1.5730 |
| 28 | O | O | $C_6H_{13}$-n | |
| 29 | O | S | $C_6H_{13}$-n | |
| 30 | S | O | $C_6H_{13}$-n | $n_D^{20}$ 1.5710 |
| 31 | S | S | $C_6H_{13}$-n | |
| 32 | O | O | $CH_2(CH_2)_2CH(CH_3)_2$ | |
| 33 | O | S | $CH_2(CH_2)_2CH(CH_3)_2$ | |
| 34 | S | O | $CH_2(CH_2)_2CH(CH_3)_2$ | |

Table 1 - Continuation

| Compound No. | $Y^1$ | $Y^2$ | R | |
|---|---|---|---|---|
| 35 | O | O | $CH_2CH_2CH-CH_2CH_3$<br>\|<br>$CH_3$ | |
| 36 | S | O | $CH_2CH_2CH-CH_2CH_3$<br>\|<br>$CH_3$ | |
| 37 | O | O | $CH_2CH-(CH_2)_2CH_3$<br>\|<br>$CH_3$ | |
| 38 | S | O | $CH_2CH-(CH_2)_2CH_3$<br>\|<br>$CH_3$ | |
| 39 | O | O | $CH_2CH_2-C(CH_3)_3$ | |
| 40 | O | S | $CH_2CH_2-C(CH_3)_3$ | |
| 41 | S | O | $CH_2CH_2-C(CH_3)_3$ | |
| 42 | S | S | $CH_2CH_2-C(CH_3)_3$ | |
| 43 | O | O | $CH_2CH-CH(CH_3)_2$<br>\|<br>$CH_3$ | |
| 44 | S | O | $CH_2CH-CH(CH_3)_2$<br>\|<br>$CH_3$ | |
| 45 | O | O | $CH_2C(CH_3)_2C_2H_5$ | |
| 46 | O | S | $CH_2C(CH_3)_2C_2H_5$ | |
| 47 | S | O | $CH_2C(CH_3)_2C_2H_5$ | |
| 48 | S | S | $CH_2C(CH_3)_2C_2H_5$ | |
| 49 | O | O | $C_7H_{15}$-n | |

Table 1 - Continuation

<table>
<tr><th>Compound No.</th><th>$Y^1$</th><th>$Y^2$</th><th>R</th><th></th></tr>
<tr><td>50</td><td>O</td><td>S</td><td>$C_7H_{15}$-n</td><td></td></tr>
<tr><td>51</td><td>S</td><td>O</td><td>$C_7H_{15}$-n</td><td>$n_D^{50}$ 1.5513</td></tr>
<tr><td>52</td><td>S</td><td>S</td><td>$C_7H_{15}$-n</td><td></td></tr>
<tr><td>53</td><td>O</td><td>O</td><td>$CH_2(CH_2)_3-CH(CH_3)_2$</td><td></td></tr>
<tr><td>54</td><td>S</td><td>O</td><td>$CH_2(CH_2)_3-CH(CH_3)_2$</td><td></td></tr>
<tr><td>55</td><td>S</td><td>S</td><td>$CH_2(CH_2)_3-CH(CH_3)_2$</td><td></td></tr>
<tr><td>56</td><td>O</td><td>O</td><td>$CH_2(CH_2)_2CH-C_2H_5$<br>|<br>$CH_3$</td><td></td></tr>
<tr><td>57</td><td>S</td><td>O</td><td>$CH_2(CH_2)_2CH-C_2H_5$<br>|<br>$CH_3$</td><td></td></tr>
<tr><td>58</td><td>O</td><td>O</td><td>$CH_2CH_2CH-(CH_2)_2CH_3$<br>|<br>$CH_3$</td><td></td></tr>
<tr><td>59</td><td>S</td><td>O</td><td>$CH_2CH_2CH-(CH_2)_2CH_3$<br>|<br>$CH_3$</td><td></td></tr>
<tr><td>60</td><td>O</td><td>O</td><td>$CH_2CH-(CH_2)_3CH_3$<br>|<br>$CH_3$</td><td></td></tr>
<tr><td>61</td><td>S</td><td>O</td><td>$CH_2CH-(CH_2)_3CH_3$<br>|<br>$CH_3$</td><td></td></tr>
<tr><td>62</td><td>O</td><td>O</td><td>$CH_2(CH_2)_2-C(CH_3)_3$</td><td></td></tr>
<tr><td>63</td><td>S</td><td>O</td><td>$CH_2(CH_2)_2-C(CH_3)_3$</td><td></td></tr>
</table>

Table 1 - Continuation

| Compound No. | $Y^1$ | $Y^2$ | R | |
|---|---|---|---|---|
| 64 | O | O | $CH_2CH_2CH-CH(CH_3)_2$<br>$CH_3$ | |
| 65 | S | O | $CH_2CH_2CH-CH(CH_3)_2$<br>$CH_3$ | |
| 66 | O | O | $CH_2CH-CH_2CH(CH_3)_2$<br>$CH_3$ | |
| 67 | S | O | $CH_2CH-CH_2CH(CH_3)_2$<br>$CH_3$ | |
| 68 | O | O | $CH_2CH_2C(CH_3)_2C_2H_5$ | |
| 69 | S | O | $CH_2CH_2C(CH_3)_2C_2H_5$ | |
| 70 | O | O | $CH_2C(CH_3)_2CH_2CH_2CH_3$ | |
| 71 | S | O | $CH_2C(CH_3)_2CH_2CH_2CH_3$ | |
| 72 | O | O | $CH_2CH_2CH-C_2H_5$<br>$C_2H_5$ | |
| 73 | S | O | $CH_2CH_2CH-C_2H_5$<br>$C_2H_5$ | |
| 74 | O | O | $CH_2CH-(CH_2)_2CH_3$<br>$C_2H_5$ | |
| 75 | S | O | $CH_2CH-(CH_2)_2CH_3$<br>$C_2H_5$ | |

Table 1 - Continuation

| Compound No. | $Y^1$ | $Y^2$ | R | |
|---|---|---|---|---|
| 76 | O | O | $CH_2CH(C_2H_5)-CH(CH_3)_2$ | |
| 77 | S | O | $CH_2CH(C_2H_5)-CH(CH_3)_2$ | |
| 78 | O | O | $C_8H_{17}$-n | |
| 79 | S | O | $C_8H_{17}$-n | |
| 80 | O | O | $CH_2(CH_2)_4-CH(CH_3)_2$ | |
| 81 | S | O | $CH_2(CH_2)_4-CH(CH_3)_2$ | |
| 82 | O | O | $CH_2$ 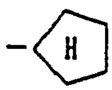 | |
| 83 | S | O | $CH_2$ 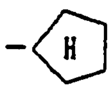 | |
| 84 | O | O | $CH_2$ 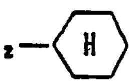 | |
| 85 | O | S | $CH_2$ 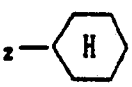 | |
| 86 | S | O | $CH_2$ 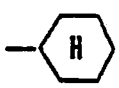 | $n_D^{50}$ 1.5630 |
| 87 | S | S | $CH_2$ 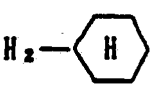 | |
| 88 | O | O | $CH_2$ 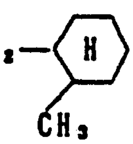 | |
| 89 | O | O | $CH_2CF_3$ | |

Table 1 - Continuation

| Compound No. | $Y^1$ | $Y^2$ | R | |
|---|---|---|---|---|
| 90 | O | S | $CH_2CF_3$ | |
| 91 | S | O | $CH_2CF_3$ | mp. 125 - 130 °C |
| 92 | S | S | $CH_2CF_3$ | |
| 93 | O | O | $CH_2CH_2CF_3$ | |
| 94 | S | O | $CH_2CH_2CF_3$ | |
| 95 | O | O | $CH_2CF_2CHF_2$ | |
| 96 | S | O | $CH_2CF_2CHF_2$ | $n_D^{22}$ 1.5498 |
| 97 | O | O | $CH_2CH_2CH_2CF_3$ | |
| 98 | S | O | $CH_2CH_2CH_2CF_3$ | $n_D^{40}$ 1.5123 |
| 99 | O | O | $CH_2CH_2OCH_3$ | |
| 100 | O | S | $CH_2CH_2OCH_3$ | $n_D^{50}$ 1.5802 |
| 101 | S | O | $CH_2CH_2OCH_3$ | |
| 102 | S | S | $CH_2CH_2OCH_3$ | |
| 103 | O | O | $CH_2CH_2OC_2H_5$ | |
| 104 | S | O | $CH_2CH_2OC_2H_5$ | $n_D^{22}$ 1.5810 |
| 105 | O | O | $CH_2CH_2O(CH_2)_2CH_3$ | |
| 106 | S | O | $CH_2CH_2O(CH_2)_2CH_3$ | |
| 107 | O | O | $CH_2(CH_2)_2OCH_3$ | |
| 108 | O | S | $CH_2(CH_2)_2OCH_3$ | |
| 109 | S | O | $CH_2(CH_2)_2OCH_3$ | |
| 110 | O | O | $CH_2(CH_2)_3OCH_3$ | |

Table 1 - Continuation

| Compound No. | $Y^1$ | $Y^2$ | R | |
|---|---|---|---|---|
| 111 | S | O | $CH_2(CH_2)_3OCH_3$ | |
| 112 | O | O | $CH_2(CH_2)_4OC_2H_5$ | |
| 113 | S | O | $CH_2(CH_2)_4OC_2H_5$ | |
| 114 | O | O | $CH_2(CH_2)_4O(CH_2)_4CH_3$ | |
| 115 | S | O | $CH_2(CH_2)_4O(CH_2)_4CH_3$ | |
| 116 | O | O | $CH_2COOCH_3$ | |
| 117 | S | O | $CH_2COOCH_3$ | |
| 118 | O | O | $CH_2COOC_2H_5$ | |
| 119 | O | S | $CH_2COOC_2H_5$ | |
| 120 | S | O | $CH_2COOC_2H_5$ | $n_D^{50}$ 1.5700 |
| 121 | S | S | $CH_2COOC_2H_5$ | |
| 122 | O | O | $CH_2CH_2COOC_2H_5$ | |
| 123 | O | S | $CH_2CH_2COOC_2H_5$ | |
| 124 | S | O | $CH_2CH_2COOC_2H_5$ | |
| 125 | O | O | $CH_2COO$ 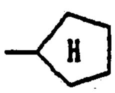 | |
| 126 | S | O | $CH_2COO$ 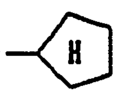 | |
| 127 | S | S | $CH_2COO$ 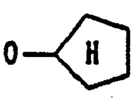 | |
| 128 | O | O | $CH_2CON(CH_3)_2$ | |
| 129 | S | O | $CH_2CON(CH_3)_2$ | |

Table 1 - Continuation

| Compound No. | $Y^1$ | $Y^2$ | R | |
|---|---|---|---|---|
| 130 | O | O | 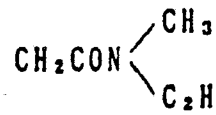 | |
| 131 | S | O | 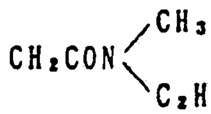 | |
| 132 | O | O | $CH_2CON(C_2H_5)_2$ | |
| 133 | O | S | $CH_2CON(C_2H_5)_2$ | |
| 134 | S | O | $CH_2CON(C_2H_5)_2$ | $n_D^{50}$ 1.5640 |
| 135 | S | S | $CH_2CON(C_2H_5)_2$ | |
| 136 | O | O | $CH_2CON(CH_2CH_2CH_3)_2$ | |
| 137 | O | S | $CH_2CON(CH_2CH_2CH_3)_2$ | |
| 138 | S | O | $CH_2CON(CH_2CH_2CH_3)_2$ | mp. 177 - 178 ℃ |
| 139 | S | S | $CH_2CON(CH_2CH_2CH_3)_2$ | |
| 140 | O | O | $CH_2CON[CH(CH_3)_2]_2$ | |
| 141 | S | O | $CH_2CON[CH(CH_3)_2]_2$ | |
| 142 | S | S | $CH_2CON[CH(CH_3)_2]_2$ | |
| 143 | O | O | $CH_2CON[(CH_2)_3CH_3]_2$ | |
| 144 | S | O | $CH_2CON[(CH_2)_3CH_3]_2$ | |
| 145 | S | S | $CH_2CON[(CH_2)_3CH_3]_2$ | |
| 146 | O | O | 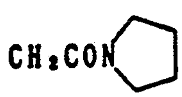 | |
| 147 | S | O | 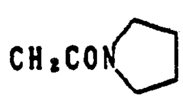 | |

Table 1 - Continuation

| Compound No. | $Y^1$ | $Y^2$ | R | |
|---|---|---|---|---|
| 148 | S | S | $CH_2CON$ 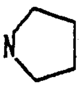 | |
| 149 | O | O | $CH_2CON$ 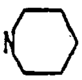 | |
| 150 | O | S | $CH_2CON$ 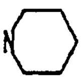 | |
| 151 | S | O | $CH_2CON$ 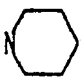 | |
| 152 | O | O | $CH_2CON$ 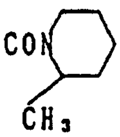 | |
| 153 | S | O | $CH_2CON$ 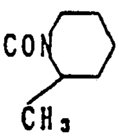 | porous crystals |
| 154 | O | S | $CH_2CON$ 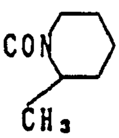 | |
| 155 | O | O | $CH_2CON$ 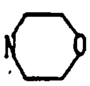 | |
| 156 | S | O | $CH_2CON$ 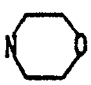 | |
| 157 | O | O | $CH_2CON$ 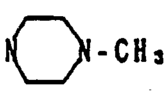 | |
| 158 | O | S | $CH_2CON$ 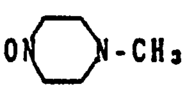 | |
| 159 | S | O | $CH_2CON$ 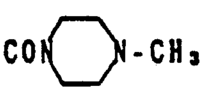 | |

Table 1 - Continuation

| Compound No. | $Y^1$ | $Y^2$ | R | |
|---|---|---|---|---|
| 160 | O | O | 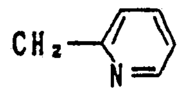 | |
| 161 | O | S | 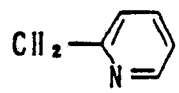 | |
| 162 | S | O | 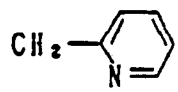 | porous crystals |
| 163 | S | S | 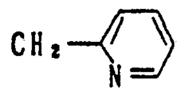 | |
| 164 | O | O | 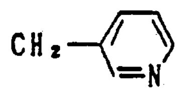 | |
| 165 | S | O | 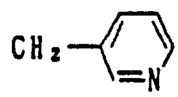 | |
| 166 | O | O | 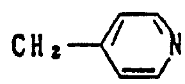 | |
| 167 | S | O | 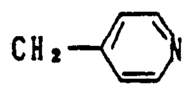 | |
| 168 | O | O | 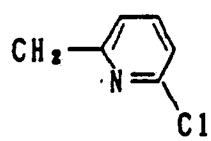 | |
| 169 | S | O | 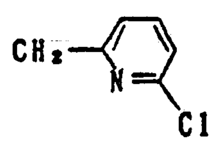 | |
| 170 | O | O | 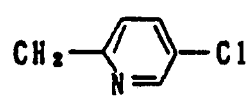 | |
| 171 | S | O | 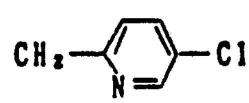 | |

Table 1 - Continuation

| Compound No. | $Y^1$ | $Y^2$ | R | |
|---|---|---|---|---|
| 172 | O | O | 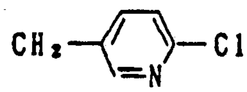 | |
| 173 | S | O | 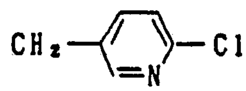 | |
| 174 | O | O | 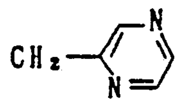 | |
| 175 | S | O | 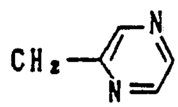 | |
| 176 | O | O | 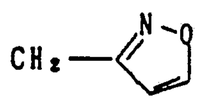 | |
| 177 | S | O | 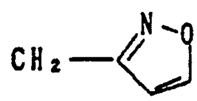 | |
| 178 | O | O | 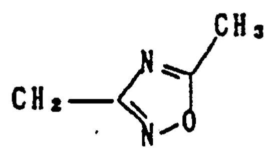 | |
| 179 | S | O | 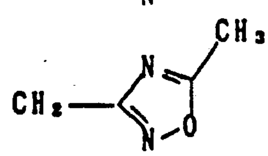 | |
| 180 | O | O | 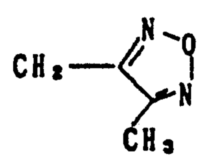 | |
| 181 | S | O | 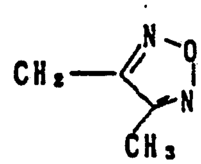 | |

Table 1 - Continuation

| Compound No. | $Y^1$ | $Y^2$ | R | |
|---|---|---|---|---|
| 182 | O | O | 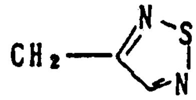 | |
| 183 | S | O | 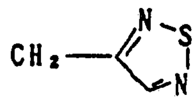 | |
| 184 | O | O | 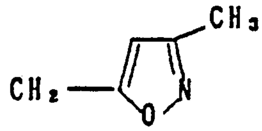 | |
| 185 | S | O | 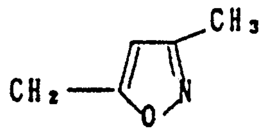 | |
| 186 | O | O | 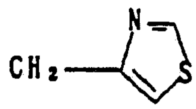 | |
| 187 | S | O | 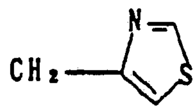 | |
| 188 | O | O | 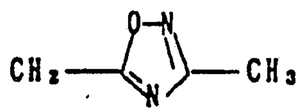 | |
| 189 | S | O | 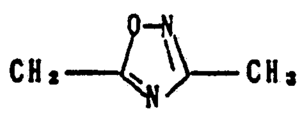 | |
| 190 | O | O | 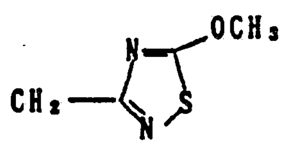 | |
| 191 | S | O | 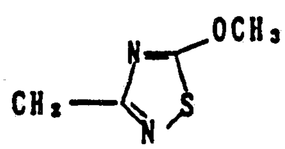 | |

Table 1 - Continuation

| Compound No. | $Y^1$ | $Y^2$ | R | |
|---|---|---|---|---|
| 192 | O | O | 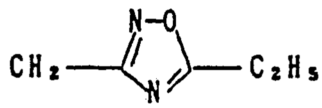 | |
| 193 | S | O | 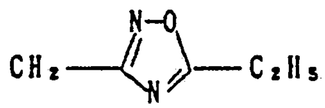 | |

Table 2

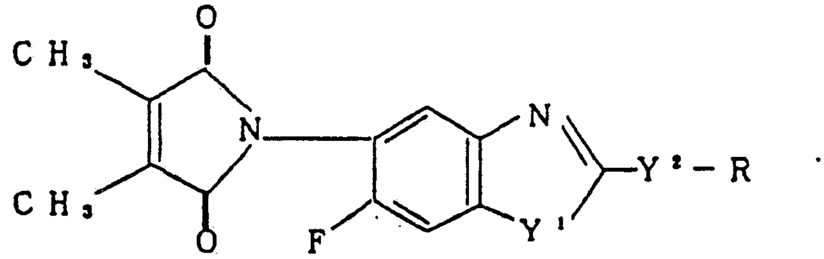

| Compound No. | $Y^1$ | $Y^2$ | R | |
|---|---|---|---|---|
| 194 | S | O | $CH_3$ | |
| 195 | S | O | $C_2H_5$ | |
| 196 | O | O | $C_2H_5$ | |
| 197 | S | O | $C_3H_7$-n | |
| 198 | S | O | $C_3H_7$-n | mp. 83 - 87 ℃ |
| 199 | S | O | $C_3H_7$-n | |
| 200 | O | S | $C_4H_9$-n | |
| 201 | S | O | $C_4H_9$-n | |
| 202 | S | O | $C_5H_{11}$-n | |
| 203 | S | S | $C_5H_{11}$-iso | |
| 204 | O | O | $C_6H_{13}$-n | |
| 205 | S | S | $CH_2CH(CH_3)-(CH_2)_2CH_3$ | |
| 206 | S | S | $CH_2CH_2C(CH_3)_3$ | |
| 207 | S | O | $C_7H_{15}$-n | |
| 208 | S | O | $C_7H_{15}$-iso | |

Table 2 - Continuation

| Compound No. | $Y^1$ | $Y^2$ | R | |
|---|---|---|---|---|
| 209 | S | O | $CH_2CH-(CH_2)_3CH_3$ with $CH_3$ on the CH | |
| 210 | S | O | $CH_2CH-CH(CH_3)_2$ with $C_2H_5$ on the CH | |
| 211 | S | O | $C_8H_{17}$-n | |
| 212 | S | O | $CH_2$ — 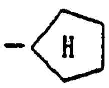 | |
| 213 | S | S | $CH_2CF_3$ | |
| 214 | S | S | $CH_2(CH_2)_2CF_3$ | |
| 215 | O | O | $CH_2CH_2OCH_3$ | |
| 216 | S | O | $CH_2CH_2OCH_3$ | |
| 217 | S | S | $CH_2COOC_2H_5$ | |
| 218 | S | O | $CH_2COO$— 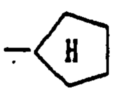 | |
| 219 | S | O | $CH_2CON(C_2H_5)_2$ | |
| 220 | O | O | $CH_2CON(C_3H_7-n)_2$ | |
| 221 | S | O | $CH_2CO$ 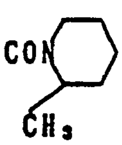 | |
| 222 | S | O | $CH_2CO$ 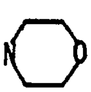 | |

Table 2 - Continuation

| Compound No. | Y¹ | Y² | R | |
|---|---|---|---|---|
| 223 | S | O | 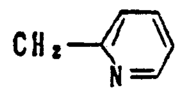 | |
| 224 | S | O | 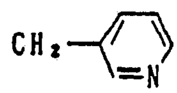 | |
| 225 | S | O | 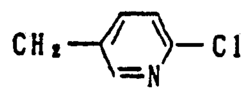 | |
| 226 | S | O | 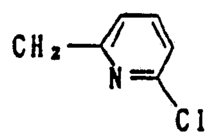 | |
| 227 | S | O | 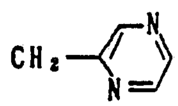 | |
| 228 | O | O | 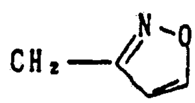 | |
| 229 | S | O | 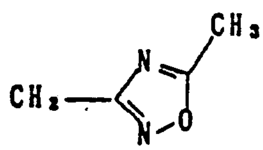 | |
| 230 | S | O | 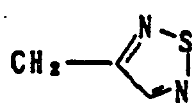 | |
| 231 | S | O | 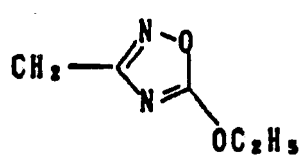 | |

Table 3

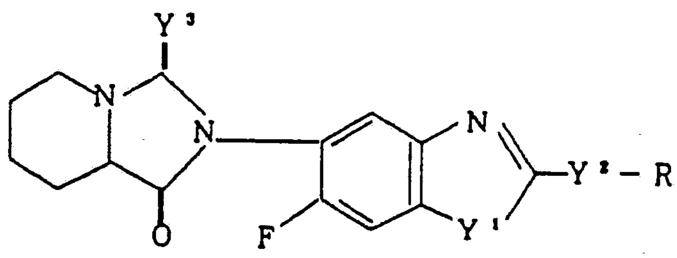

| Compound No. | $Y^1$ | $Y^2$ | $Y^3$ | R | |
|---|---|---|---|---|---|
| 232 | S | O | O | $CH_3$ | |
| 233 | O | O | S | $C_2H_5$ | |
| 234 | S | O | O | $C_2H_5$ | |
| 235 | S | S | S | $C_3H_7$-n | |
| 236 | S | O | O | $C_3H_7$-n | $n_D^{20}$ 1.5712 |
| 237 | S | O | S | $C_3H_7$-n | mp.154.5 - 155.5°C |
| 238 | S | O | O | $C_4H_9$-n | |
| 239 | S | O | S | $C_4H_9$-iso | |
| 240 | S | O | O | $C_4H_9$-iso | |
| 241 | S | O | S | $C_5H_{11}$-n | |
| 242 | S | O | O | $C_5H_{11}$-n | |
| 243 | S | O | S | $C_5H_{11}$-iso | |
| 244 | O | O | O | $CH_2CH(CH_3)C_2H_5$ | |
| 245 | O | O | S | $C_6H_{13}$-iso | |

Table 3 - Continuation

| Compound No. | Y[1] | Y[2] | Y[3] | R | |
|---|---|---|---|---|---|
| 246 | O | O | O | $CH_2CH_2CHC_2H_5$<br>$CH_3$ | |
| 247 | O | S | S | $CH_2CH-CH(CH_3)_2$<br>$CH_3$ | |
| 248 | O | O | O | $C_6H_{13}$-iso | |
| 249 | S | O | S | $CH_2CH_2CH(CH_2)_2CH_3$<br>$CH_3$ | |
| 250 | O | O | S | $CH_2CH(CH_2)_3CH_3$<br>$CH_3$ | |
| 251 | O | S | O | $CH_2CH_2CH(C_2H_5)_2$ | |
| 252 | O | S | S | $C_8H_{17}$-iso | |
| 253 | O | O | O | $CH_2$ — 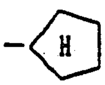 | |
| 254 | S | O | S | $CH_2$— 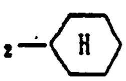 | |
| 255 | O | O | S | $CH_2CF_3$ | |
| 256 | O | O | O | $CH_2CH_2OCH_3$ | |
| 257 | S | O | S | $CH_2CH_2OC_2H_5$ | |
| 258 | S | O | O | $CH_2(CH_2)_3OCH_3$ | |
| 259 | S | O | S | $CH_2COOCH_3$ | |
| 260 | S | O | S | $CH_2COO$— 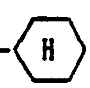 | |

Table 3 - Continuation

| Compound No. | $Y^1$ | $Y^2$ | $Y^3$ | R | |
|---|---|---|---|---|---|
| 261 | O | O | O | 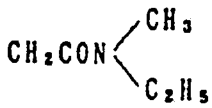 | |
| 262 | O | S | S | $CH_2CON(C_3H_7\text{-}n)_2$ | |
| 263 | S | O | S | $CH_2CON(C_4H_9\text{-}n)_2$ | |
| 264 | S | O | O | $CH_2$ 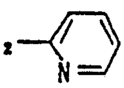 | |
| 265 | S | O | S | $CH_2$ 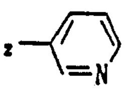 | |
| 266 | O | O | O | $CH_2$ 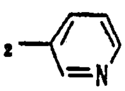 | |
| 267 | S | O | S | $CH_2$ 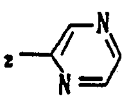 | |
| 268 | S | S | O | $CH_2$ 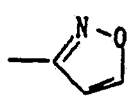 | |
| 269 | S | S | S | $CH_2$ 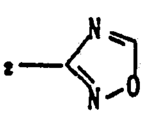 | |
| 270 | S | O | S | $CH_2$ 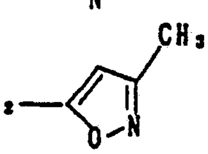 | |
| 271 | S | O | O | $CH_2$ 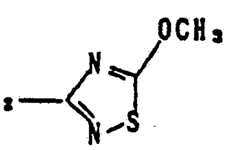 | |

Table 4

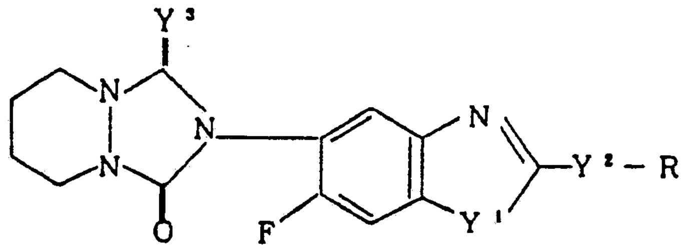

| Compound No. | $Y^1$ | $Y^2$ | $Y^3$ | R | |
|---|---|---|---|---|---|
| 272 | S | O | S | $C_2H_5$ | |
| 273 | O | O | O | $C_2H_5$ | |
| 274 | S | O | O | $C_3H_7$-n | porous crystals |
| 275 | S | O | S | $C_3H_7$-n | mp. 169 - 170°C |
| 276 | O | S | S | $C_4H_9$-n | |
| 277 | S | O | O | $C_4H_9$-n | |
| 278 | S | O | S | $C_4H_9$-n | |
| 279 | S | O | O | $C_5H_{11}$-n | |
| 280 | S | S | O | $C_6H_{13}$-iso | |
| 281 | S | O | S | $CH_2CH(CH_3)-(CH_2)_2CH_3$ | |
| 282 | S | O | S | $CH_2C(CH_3)_2C_2H_5$ | |
| 283 | S | O | S | $CH_2(CH_2)_2CH(CH_3)-C_2H_5$ | |
| 284 | S | O | O | $CH_2CH(CH_3)-(CH_2)_3CH_3$ | |

Table 4 - Continuation

| Compound No. | $Y^1$ | $Y^2$ | $Y^3$ | R | |
|---|---|---|---|---|---|
| 285 | O | S | S | $CH_2(CH_2)_2C(CH_3)_3$ | |
| 286 | O | S | O | $CH_2C(CH_3)_2CH_2CH_2CH_3$ | |
| 287 | S | S | S | 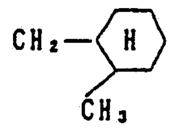 | |
| 288 | S | O | O | $CH_2CF_3$ | |
| 289 | S | O | S | $CH_2CH_2CF_3$ | |
| 290 | O | O | O | $CH_2(CH_2)_2CF_3$ | |
| 291 | S | O | S | $CH_2CH_2OCH_3$ | |
| 292 | S | S | O | $CH_2CH_2O(CH_2)_2CH_3$ | |
| 293 | S | O | S | $CH_2(CH_2)_4O(CH_2)_4CH_3$ | |
| 294 | S | O | S | $CH_2CON(CH_3)_2$ | |
| 295 | S | O | S | 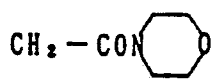 | |
| 296 | S | O | O | 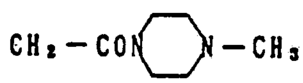 | |
| 297 | S | O | S | 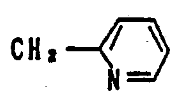 | |
| 298 | S | O | O | 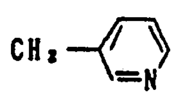 | |
| 299 | O | S | S | 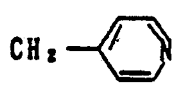 | |
| 300 | S | O | O | 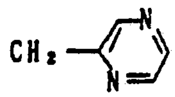 | |

Table 4 - Continuation

| Compound No. | Y[1] | Y[2] | Y[3] | R | |
|---|---|---|---|---|---|
| 301 | O | O | S | 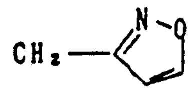 | |
| 302 | S | O | O | 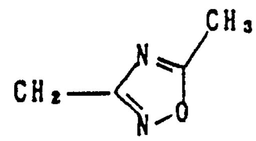 | |
| 303 | S | O | S | 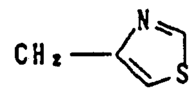 | |
| 304 | S | S | O | 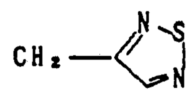 | |
| 305 | S | O | S | 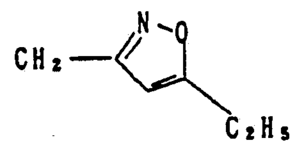 | |

Table 5

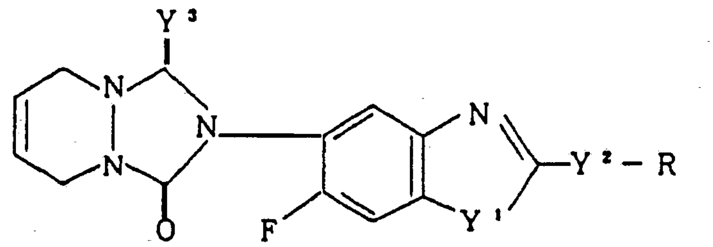

| Compound No. | $Y^1$ | $Y^2$ | $Y^3$ | R | |
|---|---|---|---|---|---|
| 306 | S | O | S | $C_2H_5$ | |
| 307 | S | S | O | $C_2H_5$ | |
| 308 | S | O | O | $C_3H_7$-n | mp. 118 - 121°C |
| 309 | S | O | S | $C_3H_7$-n | mp. 165 - 167°C |
| 310 | O | O | S | $C_3H_7$-n | |
| 311 | S | O | O | $C_4H_9$-n | |
| 312 | S | S | S | $C_4H_9$-n | |
| 313 | S | O | O | $C_4H_9$-n | |
| 314 | S | O | S | $C_5H_{11}$-n | |
| 315 | S | O | O | $C_5H_{11}$-n | |
| 316 | S | S | O | $C_5H_{11}$-iso | |
| 317 | S | S | S | $CH_2CH(CH_3)$-$C_2H_5$ | |
| 318 | S | S | O | $CH_2CH(CH_3)$-$C_2H_5$ | |
| 319 | S | O | S | $C_6H_{13}$-n | |

Table 5 - Continuation

| Compound No. | $Y^1$ | $Y^2$ | $Y^3$ | R | |
|---|---|---|---|---|---|
| 320 | S | O | O | $CH_2CH(CH_3)-(CH_2)_2CH_3$ | |
| 321 | O | O | S | $CH_2CH_2C(CH_3)_3$ | |
| 322 | S | O | O | $C_7H_{15}$-n | |
| 323 | S | S | S | $C_7H_{15}$-iso | |
| 324 | S | O | O | $CH_2(CH_2)_3CH(CH_3)-C_2H_5$ | |
| 325 | S | O | S | $CH_2(CH_2)_2C(CH_3)_3$ | |
| 326 | S | O | O | $CH_2CH_2C(CH_3)_2C_2H_5$ | |
| 327 | S | O | O | $C_8H_{17}$-n | |
| 328 | S | O | S | $C_8H_{17}$-iso | |
| 329 | S | O | O | $CH_2$ — 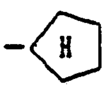 | |
| 330 | S | S | S | $CH_2$— 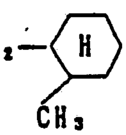 | |
| 331 | S | S | O | $CH_2CF_3$ | |
| 332 | S | O | S | $CH_2CF_3$ | |
| 333 | S | S | O | $CH_2(CH_2)_2CF_3$ | |
| 334 | S | S | S | $CH_2CH_2OCH_3$ | |
| 335 | O | S | O | $CH_2(CH_2)_4OC_2H_5$ | |
| 336 | S | S | S | $CH_2COOC_2H_5$ | |

Table 5 - Continuation

| Compound No. | $Y^1$ | $Y^2$ | $Y^3$ | R | |
|---|---|---|---|---|---|
| 337 | S | S | O | $CH_2CON(C_2H_5)_2$ | |
| 338 | S | S | S | 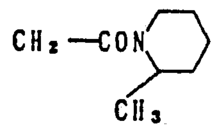 | |
| 339 | S | S | S | 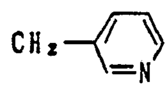 | |
| 340 | O | O | O | 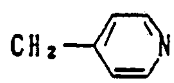 | |
| 341 | S | S | S | 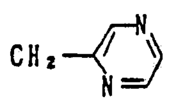 | |
| 342 | O | S | O | 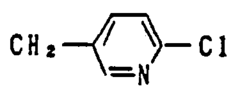 | |
| 343 | S | S | S | 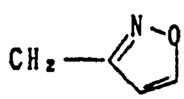 | |
| 344 | S | S | O | 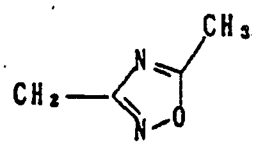 | |
| 345 | S | S | O | 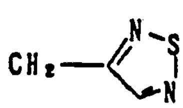 | |

The compounds represented by the general formula (II) which can be obtained by a process similar to that employed in the afore-mentioned Example 3 are demonstrated in the following Table 6 together with those obtained in Example 3.

Table 6

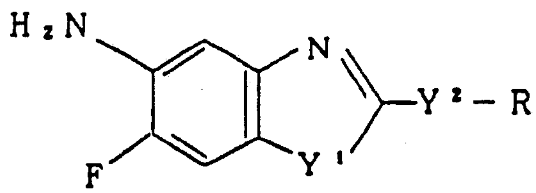

| Compound No. | $Y^1$ | $Y^2$ | R | |
|---|---|---|---|---|
| II − 1 | S | O | $CH_3$ | mp. 152 - 154°C |
| II − 2 | S | O | $C_2H_5$ | mp. 107 - 110°C |
| II − 3 | S | S | $C_2H_5$ | |
| II − 4 | O | O | $C_3H_7$-n | |
| II − 5 | O | S | $C_3H_7$-n | mp. 110 - 111°C |
| II − 6 | S | O | $C_3H_7$-n | |
| II − 7 | S | S | $C_3H_7$-n | oily |
| II − 8 | S | O | $C_4H_9$-n | mp. 101 - 103°C |
| II − 9 | O | S | $C_4H_9$-iso | |
| II −10 | S | O | $C_4H_9$-iso | mp. 95 - 98°C |
| II −11 | S | O | $C_5H_{11}$-n | oily |
| II −12 | O | O | $C_5H_{11}$-iso | |
| II −13 | S | O | $C_5H_{11}$-iso | |
| II −14 | S | O | $CH_2CH(CH_3)-C_2H_5$ | mp. 80 - 84°C |
| II −15 | S | S | $C_6H_{13}$-n | |

Table 6 - Continuation

| Compound No. | $Y^1$ | $Y^2$ | R | |
|---|---|---|---|---|
| II - 16 | S | O | $C_6H_{13}$-n | oily |
| II - 17 | S | O | $C_6H_{13}$-iso | |
| II - 18 | S | O | $CH_2CH_2C(CH_3)_3$ | |
| II - 19 | O | S | $CH_2CHCH(CH_3)_2$<br>\|<br>$CH_3$ | |
| II - 20 | O | O | $CH_2C(CH_3)_2C_2H_5$ | |
| II - 21 | S | S | $C_7H_{15}$-n | |
| II - 22 | S | O | $C_7H_{15}$-n | |
| II - 23 | S | O | $C_7H_{15}$-iso | |
| II - 24 | S | S | $CH_2CH_2CH-(CH_2)_3CH_3$<br>\|<br>$CH_3$ | |
| II - 25 | S | O | $CH_2(CH_2)_2C(CH_3)_3$ | |
| II - 26 | O | O | $CH_2CH-(CH_2)_2CH_3$<br>\|<br>$CH_3$ | |
| II - 27 | O | S | $C_8H_{17}$-n | |
| II - 28 | O | S | $C_8H_{17}$-n | |
| II - 29 | S | S | $C_8H_{17}$-iso | |
| II - 30 | S | S | $CH_2$- 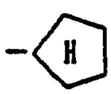 | |
| II - 31 | S | O | $CH_2$- 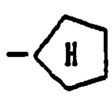 | |

Table 6 - Continuation

| Compound No. | $Y^1$ | $Y^2$ | R | |
|---|---|---|---|---|
| II-32 | O | O | 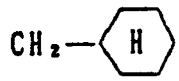 | |
| II-33 | S | O | 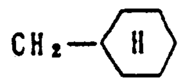 | oily |
| II-34 | S | S | 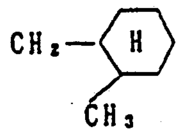 | |
| II-35 | O | O | 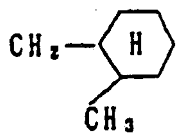 | |
| II-36 | S | S | $CH_2CF_3$ | |
| II-37 | O | O | $CH_2CF_3$ | |
| II-38 | S | O | $CH_2CF_3$ | mp. 106 - 110℃ |
| II-39 | S | S | $CH_2CH_2CF_3$ | |
| II-40 | S | O | $CH_2CH_2CF_3$ | |
| II-41 | S | O | $CH_2CF_2CF_2H$ | oily |
| II-42 | O | O | $CH_2(CH_2)_2CF_3$ | |
| II-43 | S | O | $CH_2(CH_2)_2CF_3$ | oily |
| II-44 | O | S | $CH_2CH_2OCH_3$ | mp. 100 - 105℃ |
| II-45 | S | O | $CH_2CH_2OCH_3$ | |
| II-46 | S | O | $CH_2CH_2OC_2H_5$ | oily |
| II-47 | O | O | $CH_2(CH_2)_3-OCH_3$ | |

Table 6 - Continuation

| Compound No. | $Y^1$ | $Y^2$ | R | |
|---|---|---|---|---|
| II-48 | S | O | $CH_2(CH_2)_4O(CH_2)_2CH_3$ | |
| II-49 | O | O | $CH_2COOCH_3$ | |
| II-50 | S | O | $CH_2COOCH_3$ | |
| II-51 | S | S | $CH_2COOC_2H_5$ | |
| II-52 | S | O | $CH_2COOC_2H_5$ | mp. 94 - 100℃ |
| II-53 | O | S | $CH_2CH_2COOC_2H_5$ | |
| II-54 | O | O | $CH_2CON(CH_3)_2$ | |
| II-55 | S | O | $CH_2CON(CH_3)-C_2H_5$ | |
| II-56 | S | O | $CH_2CON(C_2H_5)_2$ | oily |
| II-57 | O | O | $CH_2CON(C_3H_7\text{-}n)_2$ | oily |
| II-58 | S | O | $CH_2CON(C_3H_7\text{-}n)_2$ | |
| II-59 | S | S | 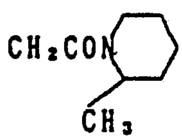 | oily |
| II-60 | S | O | 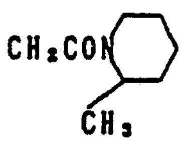 | |
| II-61 | O | O | 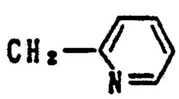 | |
| II-62 | S | O | 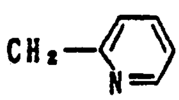 | oily |

Table 6 - Continuation

| Compound No. | $Y^1$ | $Y^2$ | R | |
|---|---|---|---|---|
| II - 63 | O | S | 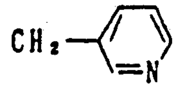 | |
| II - 64 | S | O | 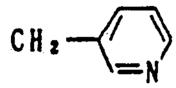 | |
| II - 65 | O | O | 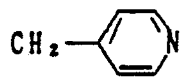 | |
| II - 66 | S | O | 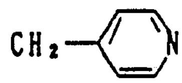 | |
| II - 67 | S | S | 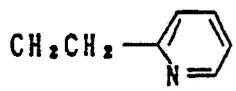 | |
| II - 68 | S | O | 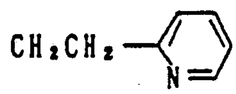 | |
| II - 69 | O | S | 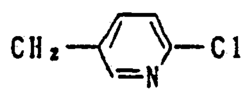 | |
| II - 70 | S | O | 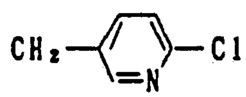 | |
| II - 71 | S | O | 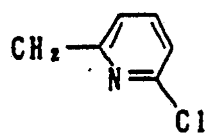 | |
| II - 72 | O | O | 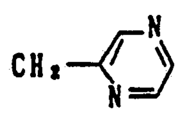 | |
| II - 73 | S | O | 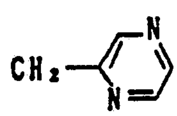 | |
| II - 74 | O | S | 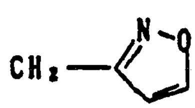 | |

Table 6 - Continuation

| Compound No. | Y¹ | Y² | R | |
|---|---|---|---|---|
| II -75 | S | O | 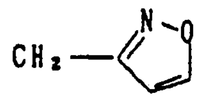 | |
| II -76 | S | O | 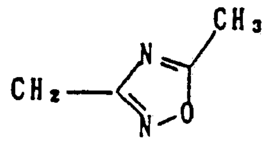 | |
| II -77 | S | S | 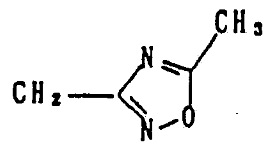 | |
| II -78 | O | O | 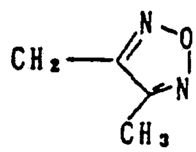 | |
| II -79 | S | O | 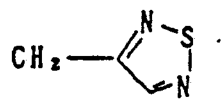 | |
| II -80 | S | O | 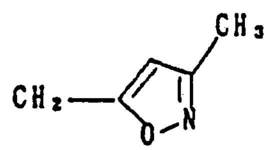 | |
| II -81 | S | O | 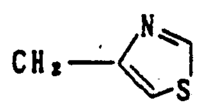 | |
| II -82 | S | O | 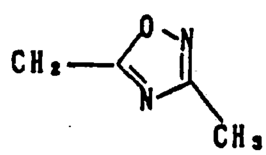 | |
| II -83 | S | S | 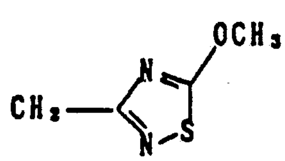 | |

Table 6 - Continuation

| Compound No. | $Y^1$ | $Y^2$ | R | |
|---|---|---|---|---|
| II-84 | S | O | 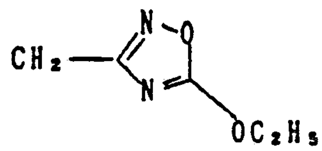 | |

Biotest Example:

Control compounds

E-1

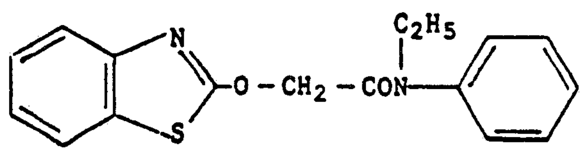

(disclosed in Japanese Patent Application Publication No. 14711-1988)

Example 4

Submerged application test against lowland weeds under flooding condition

Formulation of Active Compounds

Carrier : 5 parts by weight of acetone
Emulsifier: 1 part by weight of benzyloxy polyglycol ether

To produce a suitable preparation of active compound, 1 part by weight of active compound was mixed with the stated amount of carrier and with the stated amount of emulsifier, and the resulting emulsifiable concentrate was then diluted with water to the desired concentrations.

Test Procedures

Each of several pots, having a size of 25 x 20 x 9 cm and an area of 1/2,000 are, was filled with soil taken out from a paddy field. Young paddy-rice plants (Nihonbare Variety) of the 2.5-leaf stage, with a height of 15 cm, were transplanted into these pots. Each pot has two zones, with the number of the plants grown in each zone being three. Then, seeds of the following weeds were sown in the soil, which was kept under wet conditions:
barnyard grass (Echinochloa crus-galli);
umbrella plant (Cyperus diformis);

monchoria (Monochoria vaginalis); and
annual broad-leaved weeds such as false pimpernel (Lindernia pyxidaria), toothcup (Rotala indica), American waterwort (Elatine triandra), red stem (Ammannia multiflora), and dopatrium (Dopatrium).

After 2 days, such an amount of water was supplied into each of the pots that a sheet of water with a thickness of 2 - 3 cm, was formed over the soil in each pot. Five days after the transplantation of the rice plants, the emulsion of the active compound, which had been prepared in the manner mentioned above, was applied to the pots in a predetermined amount by means of a pipette. After that, the water sheet was kept in a thickness of about 3 cm.

Four weeks after the application of the active compound, the degree of damage to the weeds and the degree of phytotoxicity on the paddy-rice plants were determined, and recorded according to the following assessment scale rated from 0 to 5.

| Rating | Herbicidal effect of active compound on weed in % * |
|---|---|
| 5 | 95% or more (fatal effect) |
| 4 | at least 80% and less than 95% |
| 3 | at least 50% and less than 80% |
| 2 | at lease 30% and less than 50% |
| 1 | at least 10% and less than 30% |
| 0 | less than 10% (no herbicidal effect) |
| **Rating** | **Phytotoxic effect of active compound on crops in % *** |
| 5 | at least 90% (fatal phytotoxicity) |
| 4 | at least 50% and less than 90% |
| 3 | at least 30% and less than 50% |
| 2 | at least 10% and less than 30% |
| 1 | more than 0% and less than 10% |
| 0 | 0% (no phytotoxicity) |

* These values (%) are those obtained by comparing the test data in the treated section with the test data in the control (untreated) section.

The test results are shown in Table 7.

Table 7

| Active compound No. | Amount of active compound (kg/ha) | Herbicidal effect on weeds | | | | Phytotoxic effect on rice plants |
|---|---|---|---|---|---|---|
| | | Barnyard grass | Umbrella plant | Monochoria | Annual broad-leaved grass | |
| 2 | 0.25 | 4 | 5 | 5 | 5 | 0 |
| 14 | 0.25 | 4 | 5 | 5 | 5 | 1 |
| 91 | 0.25 | 5 | 5 | 5 | 5 | 0 |
| 198 | 0.25 | 4 | 5 | 5 | 5 | 0 |
| 237 | 0.25 | 4 | 5 | 5 | 5 | 0 |
| 275 | 0.25 | 4 | 5 | 5 | 5 | 1 |
| Control E-1 | 0.25 | 1 | 2 | 1 | 1 | 0 |

Example 5

Pre-emergence soil treatment against upland weeds

In a greenhouse, a number of pots, each having an area of 500 cm², were charged with soil obtained from a cultivated field. Seeds of soy bean were sown into the soil in the pots. Thereafter the surface of the soil was covered with a soil layer. The soil layer contained seeds of fingergrass (Digitaria), wild blite (Amaranthus) and goosefoot (Chenopodium album). The thickness of the soil layer was about 1 cm.

One day after the sowing, a predetermined amount of the preparation of the active compound, which had been produced in the same manner as in Example 4, was uniformly applied to the top soil layer in each pot.

Four weeks after the application of the active compound, the degree of damage to the weeds and the degree of phytotoxicity on the crops were determined in the same manner as in Example 4. The test results are shown in Table 8.

Table 8

| Active compound No. | Amount of active compound (kg/ha) | Herbicidal effect on weeds | | | Phytotoxic effect on soy bean plants |
|---|---|---|---|---|---|
| | | Fingergrass | Wild blite | Goosefoot | |
| 2 | 0.5 | 3 | 5 | 5 | 0 |
| 91 | 0.5 | 4 | 5 | 5 | 0 |
| 100 | 0.5 | 4 | 5 | 5 | 0 |
| 138 | 0.5 | 4 | 5 | 5 | 0 |
| Control E-1 | 0.5 | 1 | 0 | 0 | 0 |

Example 6

Herbicidal test by foliage application on upland weeds

In a greenhouse, a number of pots, each having an area of 500 cm², were charged with soil obtained from a cultivated field. Seeds of wheat (Triticum sativium Linuaeus) were sown onto the soil in the pots. Thereafter, the surface of the soil was covered with a soil layer.

The soil layer contained seeds of fingergrass (Digitaria), wild blite (Amaranthus) and goosefoot (Chenopodium album). The thickness of the soil layer was about 1 cm.

The wheat plants were grown for 14 days, and then the plants were uniformly sprayed with a predetermined amount of the formulation of the active compound which had been produced in the same manner as in Example 4.

Four weeks after the application of the active compound, the degree of damage to the weeds and the degree of phytotoxicity on the wheat plants were determined in the same manner as in Example 4. The test results are shown in Table 9.

Table 9

| Active compound No. | Amount of active compound (kg/ha) | Herbicidal effect on weeds | | | Phytotoxicity on wheat plants |
|---|---|---|---|---|---|
| | | Fingergrass | Wild blite | Goosefoot | |
| 10 | 0.25 | 4 | 5 | 5 | 0 |
| 14 | 0.25 | 5 | 5 | 5 | 1 |
| 104 | 0.25 | 4 | 5 | 5 | 0 |
| 138 | 0.25 | 4 | 5 | 5 | 0 |
| 237 | 0.25 | 4 | 5 | 5 | 0 |
| Control E-1 | 0.5 | 1 | 0 | 0 | 0. |

## Claims

1) Benzazoles of the formula (I)

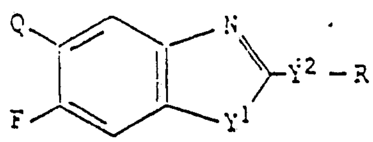

wherein Q represents

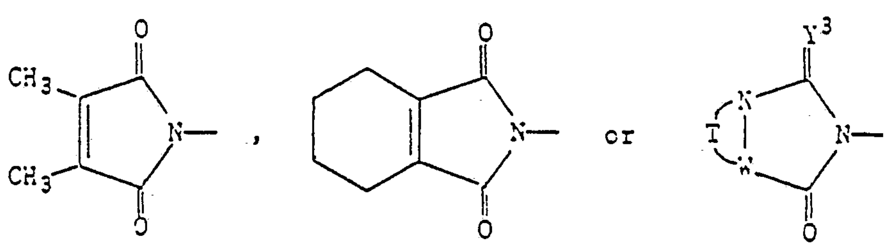

wherein
T represents $-(CH_2)_4-$ or $-CH_2-CH=CH-CH_2-$,
W represents CH or N,
$Y^3$ represents O or S,
$Y^1$ and $Y^2$ each independently represent O or S, and
R represents an optionally halogen substituted $C_{1-10}$ alkyl group, a cycloalkylmethyl group having $C_{3-6}$ cycloalkyl which is optionally substituted by $C_{1-3}$ alkyl, or represents $C_{3-10}$ alkoxyalkyl, $-CH_2COOR^1$ or $CH_2CONR^2R^3$, wherein $R^1$ represents a $C_{1-6}$ alkyl group or a $C_{5-6}$ cycloalkyl group, $R^2$ and $R^3$ each represent a $C_{1-6}$ alkyl group or $R^2$ and $R^3$ together may form a five- or six-membered ring that may comprise oxygen or $N-CH_3$, or
R represents a methyl group substituted by a five-or six-membered heterocyclic group, wherein said heterocyclic group may be substituted with halogen, $C_{1-3}$ alkyl and/or $C_{1-3}$ alkoxy.

2) The compounds of claim 1, wherein Q represents

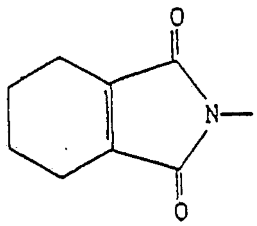

or

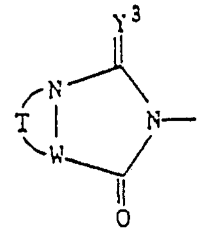

,

in which
T represents $-(CH_2)_4-$,
W represents CH or N,
$Y^3$ represents O or S,
$Y^1$ represents S, $Y^2$ represents O, and
R represents an optionally halogen substitutable $C_{1-6}$ alkyl group a cycloalkylmethyl group having optionally by methyl-substituted $C_{3-6}$ cycloalkyl, a $C_{1-3}$ alkoxy-$C_{2-3}$ alkyl group, $-CH_2COOR^1$ or $CH_2CONR^2R^3$, wherein $R^1$ represents a $C_{1-4}$ alkyl group or cyclopentyl group,
$R^2$ and $R^3$ each represent a $C_{1-4}$ alkyl group or $R^2$ and $R^3$ together may form a five- or six-membered ring that may comprise oxygen or $N-CH_3$, or
R represents a methyl group substituted by a five-membered heterocyclic group that may be substituted with chlorine, methyl or methoxy, wherein said heterocyclic group may comprise one to two nitrogen atoms and further one of oxygen, sulfur or nitrogen, or by a six-membered heterocyclic group that may comprise one to two nitrogen atoms and optionally chlorine substituent.

3) The compounds of claims 1 and 2, wherein Q represents

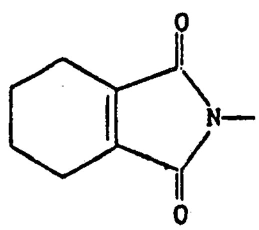

or

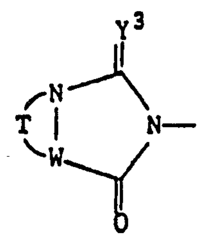

in which T represents $-(CH_2)_4-$, W represents CH or N and
$Y^3$ represents O or S,
$Y^1$ represents S, $Y^2$ represents O and
R represents methyl, ethyl, in each case straight chain or branched propyl, butyl, pentyl, hexyl or heptyl, which are optionally substituted by fluorine, methoxymethyl, methoxyethyl, ethoxymethyl, ethoxyethyl, methoxycarbonylmethyl, ethoxycarbonylmethyl, dimethylaminocarbonylmethyl, diethylaminocarbonylmethyl or dipropylaminocarbonylmethyl.

4) A compound according to claim 1, wherein such compound is 6-fluoro-2-propoxy-5-(4,5,6,7-tetrahydro-2H-isoindole-1,3-dion-2-yl)benzothiazole of the formula

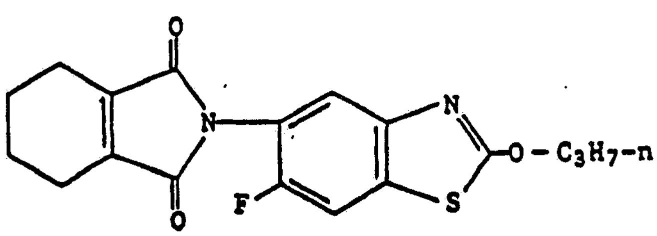

2-butoxy-6-fluoro-5-(4,5,6,7-tetrahydro-2H-isoindole-1,3-dion-2-yl)benzothiazole of the formula

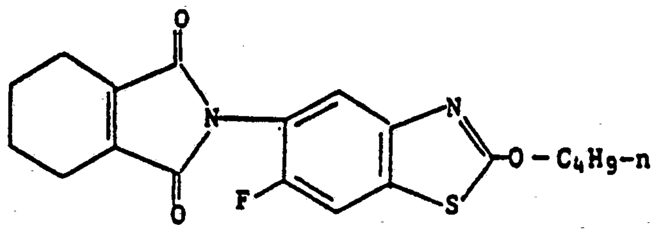

6-fluoro-2-pentoxy-5-(4,5,6,7-tetrahydro-2H-iso-indole-1,3-dion-2-yl)benzothiazole of the formula

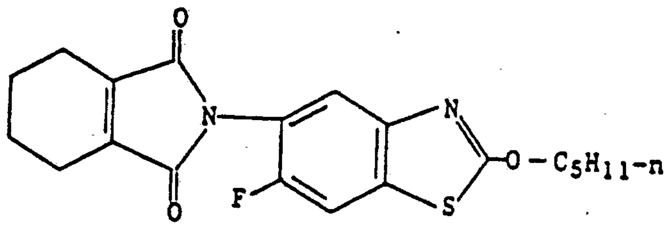

and

6-fluoro-2-propoxy-5-(3,4-tetramethylene-imidazoline-2-thion-5-on-1-yl)benzothiazole of the formula

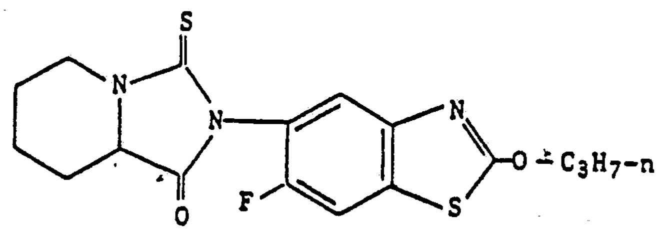

5. Process for the preparation of benzazoles of the formula (I)

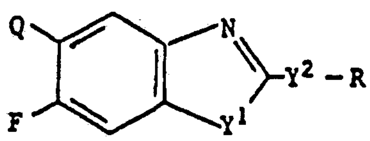

(I)

wherein Q represents

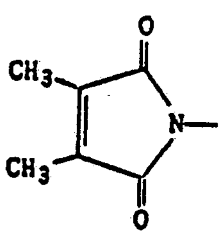 , 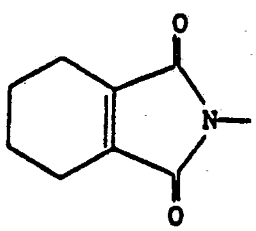 or 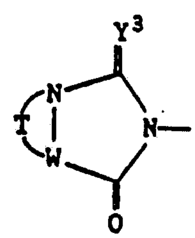

wherein
T represents $-(CH_2)_4-$ or $-CH_2-CH=CH-CH_2-$,
W represents CH or N,
$Y^3$ represents O or S,
$Y^1$ and $Y^2$ each independently represent O or S, and
R represents an optionally halogen substituted $C_{1-10}$ alkyl group, a cycloalkylmethyl group having $C_{3-6}$ cycloalkyl which is optionally substituted by $C_{1-3}$ alkyl, or represents $C_{3-10}$ alkoxyalkyl, $-CH_2COOR^1$ or

$CH_2CONR^2R^3$, wherein $R^1$ represents a $C_{1-6}$ alkyl group or a $C_{5-6}$ cycloalkyl group, $R^2$ and $R^3$ each represent a $C_{1-6}$ alkyl group or $R^2$ and $R^3$ together may form a five- or six-membered ring that may comprise oxygen or $N\text{-}CH_3$, or a five- or six-membered heterocyclic group, wherein said heterocyclic group may be substituted with halogen, $C_{1-3}$ alkyl and/or $C_{1-3}$ alkoxy,
characterized in that

a) in the case where Q represents

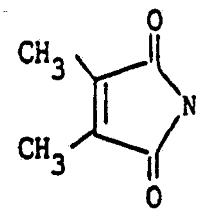 or 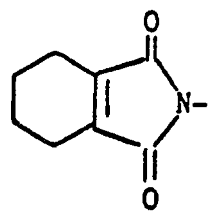 , then

Q is replaced by 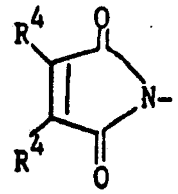 , in which

$R^4$ is methyl or tetramethylene;
compounds of the formula (II)

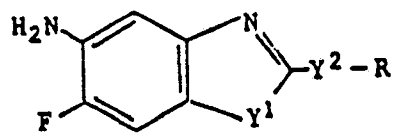

(II).

wherein $Y^1$, $Y^2$ and R are abovementioned, are reacted with compound of the formula (III)

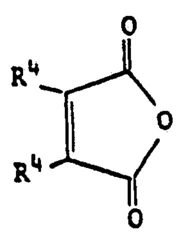

(III)

wherein $R^4$ is abovementioned, in the presence of inert solvents, or

b) in the case where Q is

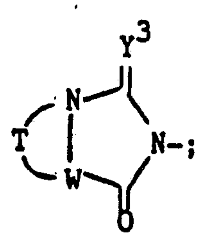

compounds of the formula (IV)

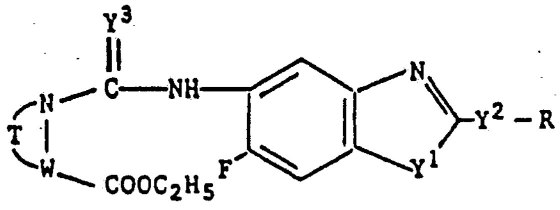

(IV)

wherein $Y^1$, $Y^2$, R, T, W and $Y^3$ are abovementioned, are condensed in the presence of inert solvents.

5. Herbicidal compositions, characterised in that they contain at least one benzazoles of the formula (I) according to claim 1.

6. Process for combating weeds, characterised in that benzazoles of the formula (I) according to claim 1 are allowed to act on weeds and/or their habitat.

7. Use of benzazoles of the formula (I) according to claim 1 for combating weeds.

8. Process for the preparation of herbicidal compositions, characterized in that benzazoles of the formula (I) according to claim 1 are mixed with extenders and/or surface active agents.

9. Compounds of the formula (IV)

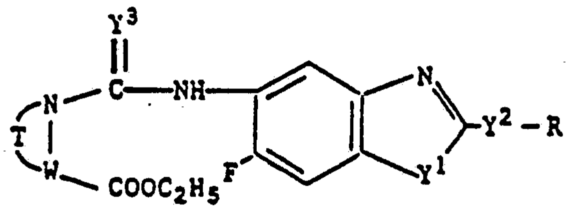

(IV)

wherein
T represents $-(CH_2)_4-$ or $-CH_2-CH=CH-CH_2-$,
W represents CH or N,
$Y^3$ represents O or S,
$Y^1$ and $Y^2$ each independently represent O or S, and
R represents an optionally halogen substituted $C_{1-10}$ alkyl group, a cycloalkylmethyl group having $C_{3-6}$ cycloalkyl which is optionally substituted by $C_{1-3}$ alkyl, or represents $C_{3-10}$ alkoxyalkyl, $-CH_2COOR^1$ or $CH_2CONR^2R^3$, wherein $R^1$ represents a $C_{1-6}$ alkyl group or a $C_{5-6}$ cycloalkyl group, $R^2$ and $R^3$ each represent a $C_{1-6}$ alkyl group or $R^2$ and $R^3$ together may form a five-or six-membered ring that may comprise oxygen or $N-CH_3$, or
R represents a methyl group substituted by a five- or six-membered heterocyclic group, wherein said heterocyclic group may be substituted with halogen, $C_{1-3}$ alkyl and/or $C_{1-3}$ alkoxy.

10. Compounds of the formula (V)

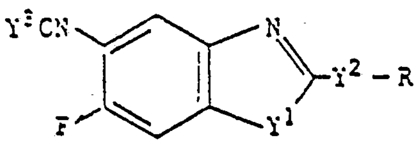

(V)

in which
$Y^1$ and $Y^2$ each independently represent O or S,
$Y^3$ represents O or S and
R represents an optionally halogen substituted $C_{1-10}$ alkyl group, a cycloalkylmethyl group having $C_{3-6}$ cycloalkyl which is optionally substituted by $C_{1-3}$ alkyl, or represents $C_{3-10}$ alkoxyalkyl, $-CH_2COOR^1$ or $-CH_2CONR^2R^3$ wherein
$R^1$ represents a $C_{1-6}$ alkyl group or a $C_{5-6}$ cycloalkyl group or
$R^2$ and $R^3$ each represent a $C_{1-6}$ alkyl group or $R^2$ and $R^3$ together may form a five- or six-membered ring that may comprise oxygen or $N-CH_3$; or
R represents a methyl group substituted by a five-or six-membered heterocyclic group, wherein said heterocyclic group may be substituted with halogen, $C_{1-3}$ alkyl and/or $C_{1-3}$ alkoxy.